# EUROPÄISCHE PATENTSCHRIFT

(11) **EP 1 495 025 B1**
(45) Veröffentlichungstag und Bekanntmachung des Hinweises auf die Patenterteilung: **20.12.2006**
(21) Anmeldenummer: 03745787.6
(22) Anmeldetag: 04.04.2003
(51) Int. Cl.: C07D 487/22, C07D 498/22, C07D 513/22, C09B 67/00

(54) **CYCLISCHE VERBINDUNGEN UND IHRE VERWENDUNG ALS LICHTABSORBER ODER LICHTEMITTER**
CYCLIC COMPOUNDS AND THE USE THEREOF AS LIGHT ABSORBERS OR LIGHT EMITTERS
COMPOSES CYCLIQUES ET LEUR UTILISATION EN TANT QU'ABSORBEURS DE LUMIERE OU EMETTEURS DE LUMIERE

(30) Priorität: 04.04.2002 DE 10214937
(43) Veröffentlichungstag der Anmeldung: 12.01.2005
(73) Patentinhaber: BASF Aktiengesellschaft, 67056 Ludwigshafen (DE)
(72) Erfinder: KÖNEMANN, Martin, 68163 Mannheim (DE); GESSNER, Thomas, 69120 Heidelberg (DE); SENS, Rüdiger, 67069 Ludwigshafen (DE); LENNARTZ, Christian, 53757 Sankt Augustin (DE); SEYBOLD, Günther, 67141 Neuhofen (DE)
(74) Vertreter: Féaux de Lacroix, Stefan
(86) Internationale Anmeldenummer: PCT/EP2003/003538
(87) Internationale Veröffentlichungsnummer: WO 2003/084960

(56) Entgegenhaltungen:
- US-A- 3 481 945
- US-A- 5 180 821
- TAUER E: "Preparation of new cyclic quaterbenzoxazole and -imidazole derivatives" SYNTHESIS, GEORG THIEME VERLAG. STUTTGART, DE, Nr. 6, 1. Mai 2002 (2002-05-01), Seiten 723-725, XP002239393 ISSN: 0039-7881

## Beschreibung

Die vorliegende Erfindung betrifft cyclische Verbindungen, Verfahren zu ihrer Herstellung, ihre Verwendung als photoaktive Effektstoffe, wie Lichtabsorber, lichtemittierende Verbindungen, Dispergiermittel oder als Komplexliganden, und diese enthaltende Komplexe.

Lichtabsorbierende Verbindungen (Lichtabsorber) im Sinne der vorliegenden Erfindung werden üblicherweise nach dem Frequenzbereich eingeteilt, in dem sie Licht absorbieren. Man unterscheidet so UV-Absorber, die UV-Licht absorbieren, Vis-Absorber (Farbmittel), die sichtbares Licht absorbieren, und IR-Absorber, die Infrarotstrahlung absorbieren. Ferner werden Lichtabsorber aufgrund ihrer Löslichkeit oder Unlöslichkeit im Anwendungsmedium und nach der Art der Abgabe der absorbierten Energie, beispielsweise als Wärme oder als Strahlung, eingeteilt und unterschieden.

Im UV-Bereich absorbierende lösliche und unlösliche Verbindungen, die die aufgenommene Energie in Form von Wärme abgeben, werden häufig als UV-Absorber zum Zweck des UV-Schutzes eingesetzt. Als Chromophore werden üblicherweise für diese Anwendungen Derivate von Triazin, Benzophenonen, Benzotriazolen, Cyanacrylaten, wie auch ZnO und TiO₂ eingesetzt. Wird die UV-Strahlung in Form von Fluoreszenzstrahlung abgegeben, was in der Regel nur bei im Anwendungsmedium löslichen Verbindungen der Fall ist, so erhält man optische Aufheller, die weiße Materialien weniger gelb erscheinen lassen. Als Chromophore für diese Anwendungen werden insbesondere Benzoxazole, Coumarine und Naphthylimide eingesetzt, vergleiche G. Pritchard, Plastic Additives, Chapmann & Hall, Weinheim 1998.

Im sichtbaren Bereich des Lichts absorbierende, im Anwendungsmedium lösliche Verbindungen, die ihre absorbierte Energie in Form von Wärme abgeben, werden als Farbstoffe bezeichnet. Geben diese im Anwendungsmedium löslichen Verbindungen Energie in Form von Strahlung ab, so spricht man von Fluoreszenzfarbstoffen. Im sichtbaren Bereich absorbierende Verbindungen, die im Anwendungsmedium unlöslich sind, werden als Pigmente bezeichnet und geben ihre Energie in Form von Wärme ab. Pigmente und Fluoreszenzfarbstoffe werden zum Einfärben von Kunststoffen, Papierfasern, Fasern usw. eingesetzt. Zu diesem Zweck werden üblicherweise Perylenverbindungen, Phthalocyaninverbindungen, Indanthronverbindungen, Azoverbindungen, Chinophthalonverbindungen, Chinacridonverbindungen, Isoindolinverbindungen, Diketopyrrolopyrrolverbindungen eingesetzt, vergleiche W. Herbst, K. Hunger, Industrial Organic Pigments, VCH Weinheim, 1993.

Bei allen photoaktiven Verbindungen sind die Anwendungsmöglichkeiten durch ihre Langzeitstabilität beschränkt. Die Langzeitstabilität drückt sich in der Beständigkeit gegenüber Licht oder UV-Strahlung, Feuchtigkeit und Wärme aus. Diese Langzeitstabilität geht meistens mit einer chemischen Stabilität einher.

Auf dem Gebiet der Komplexliganden werden als multidentale Komplexbildner häufig sogenannte Kronenether eingesetzt. Es handelt sich dabei um eine Klasse von planar gebauten makrocyclischen Polyethem. Dabei sind häufig die Sauerstoffatome durch Ethylenbrücken verbunden, wobei vielfach ein oder mehrere Benzol- oder Cyclohexanringe ankondensiert sind. Die Sauerstoffatome der Kronenether können dabei auch teilweise oder ganz durch andere Heteroatome wie Stickstoff, Phosphor oder Schwefel ersetzt sein. Hierdurch erhält man beispielsweise Aza-, Phospha- oder Thia-Kronenether. Ferner können polare Gruppen vorliegen, die die Donor-Position übernehmen können.

Die bislang bekannten Kronenether weisen nicht für alle Komplexierungsaufgaben ein durchweg geeignetes Eigenschaftsprofil auf. Es besteht daher weiterhin Nachfrage nach cyclischen Komplexliganden, die neue Eigenschaftsprofile zeigen.

In Synthesis 2002, No. 6, Seiten 723 bis 725 sind insbesondere Quaterbenzoxazol-Verbindungen und Quaterbenzimidazolverbindungen beschrieben, die als Komplexliganden eingesetzt werden können.

In der US 5,180,821 sind ein cyclisches Tetrabenzimidazol und ein Verfahren zu seiner Herstellung beschrieben. Die Herstellung erfolgt durch stufenweisen Aufbau eines linearen Tetramers über Schutzgruppen-Chemie. Das lineare Tetramer wird sodann cyclisiert. Ferner ist ein Kupferkomplex des cyclischen Tetrabenzimidazol-Produktes beschrieben. Darüber hinaus wird in der Schrift auf die ältere US 3,481,945 verwiesen, wobei das dort beschriebene Verfahren jedoch zu Fluoridin führte und nicht, wie angegeben, zu cyclischem Tetrabenzimidazol.

Organische lichtemittierende Verbindungen werden vielfach in organischen lichtemittierenden Dioden (OLED) eingesetzt. Organische Leuchtdioden (OLED) sind allgemein bekannt und beispielsweise in Angew. Chem. 1998, 110, Seiten 416 bis 443 beschrieben. Der Aufbau von organischen Leuchtdioden wurde beispielsweise von C. W. Tang und S. A. Van Slyke, Appl. Phys. Lett. 51, 913 - 915, 1987 und von M. A. Baldo, M. E. Thompson und S. R. Forrest, Pure Appl. Chem. 71, 2095-2106, 1999 beschrieben. Sie sind in vielen Gebieten anwendbar, beispielsweise in monochromen, mehr- und vollfarbigen Bildschirmen, die wiederum beispielsweise in Automobilen, Mobiltelefonen oder Notebooks Anwendung finden.

Die bekannten lichtemittierenden Materialien für OLEDs, insbesondere blaue und rote Emitter, weisen noch eine unzureichende Langzeitstabilität auf. Die Lichtemission der Emittermaterialien kann auf Fluoreszenz oder Phosphoreszenz beruhen. Phosphoreszierende Emitter sind beispielsweise in WO 01/08230 beschrieben. Sie beruhen auf Schwermetallkomplexen, die eine kurzlebige Phosphoreszenz aufweisen.

Die Verwendung von Cobaltphthalocyanin-basierten Lichtabsorbern zur optischen Datenspeicherung wird in DE-A-101 24 585 beschrieben.

### Synergisten zur Pigmentdispergierung

EP-A 0554971 beschreibt die Verwendung von sulfonierten Phthalocyaninen zur Verhinderung der Flockulation fein verteilter Phthalocyanin-Pigmente. DE-A 43 25 247 beschreibt carboxylierte und sulfonierte Perylenderivate in Kombination mit basischen Polymeren zur Verhinderung von Flockulation und zur Verbesserung rheologischer Eigenschaften in hochpigmentierten Lacksystemen. Es werden damit sulfonierte Pigmentderivate zur Oberflächenmodifizierung der Pigmente verwendet, welche dann je nach Formulierung bessere rheologische Eigenschaften und eine verbesserte Transparenz aufweisen. Weitere Erläuterungen sind in DE-A 10303916 und der dort zitierten Literatur, insbesondere in der Druckschrift Science and Technology of Pigment Dispersions A four day post graduatate intensive course presented by Institute of Materials Science zu finden.

Um die Farbeigenschaften der oberflächenmodifizierten Pigmente nicht zu verändern, benutzt man zur Oberflächenmodifikation von Perylenpigmenten (rot) rote sulfonierte Pigmentderivate, beziehungsweise zur Oberflächenmodifizierung von blauen Pigmenten, wie z.B. Phthalocyaninen (blau) oder Indanthronpigmenten (blau) blaue sulfonierte Pigmentderivate. Daher braucht man verschiedene sulfonierte Pigmentderivate für die unterschiedlich farbigen Pigmente.

Es bestand die Aufgabe, ein farbloses sulfoniertes Pigmentderivat zu finden, welches unabhängig von der Farbe des zu modifizierenden Pigments eingesetzt werden kann.

Das gelingt mit den erfindungsgemäßen sulfonierten Makrocyclen, wie beispielsweise den Cycloquaterbenzoaxazolen, Cycloquaternaphthoxazolen, Cycloquaterbenzimidazolen und Cycloquaternaphthimidazolen, welche im sichtbaren Bereich des Spektrums praktisch nicht absorbieren.

Aufgabe der vorliegenden Erfindung ist die Bereitstellung von cyclischen Verbindungen, die insbesondere als photoaktive Effektstoffe wie Lichtabsorber, Lichtemitter, oder als Komplexliganden eingesetzt werden können.

Die Aufgabe wird erfindungsgemäß gelöst durch Verwendung von cyclischen Verbindungen der allgemeinen Formel (I) 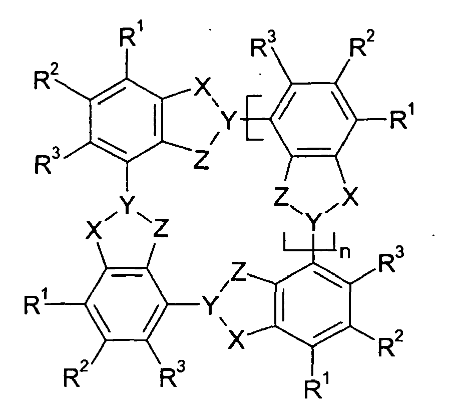 mit der Bedeutung
- n: Zahl im Bereich von 1 bis 7, vorzugsweise 1 bis 3, insbesondere 1,
- X-Y-Z: jeweils unabhängig O-C=N, N=C-O, NR⁵-C=N, N=C-NR⁵, N⁺R⁵₂-C=N, N=C-N⁺R⁵₂, O-C=N⁺R⁵, N⁺R⁵=C-O, S-C=N⁺R⁵, N⁺R⁵=C-S, S-C=N, N=C-S,
- R¹, R², R³: jeweils unabhängig H oder Substituent der Gruppe C₁₋₁₂-Alkyl, C₁₋₁₂-Alkanoyl, C₃₋₇-Cycloalkyl, C₆₋₁₂-Aryl, C₇₋₁₃-Aralkyl, C₇₋₁₃-Alkaryl, C₁₋₁₂-Alkoxy, C₆₋₁₂-Aryloxy, C₁₋₁₂-Hydroxyalkyl, Heterocyclus, C₆₋₁₂-Aroyl, die jeweils substituiert sein können, Hydroxy, Thiol, Halogen, Cyan, Isocyan, Nitro, Ammonium, Amino, Phosphin, Phosphinoxid, Sulfonsäure oder Derivat davon, Carbonsäure oder Derivat davon, Derivate des Siliziums, C₂₋₁₂-Alkinyl oder C₂₋₁₂-Alkenyl, wobei die Doppel- oder Dreifachbindungen direkt an das Cycloquatergerüst gebunden sein können oder in der Kette stehen können, Carbamate der Formel -NH-CO-OR⁷, substituierte Harnstoffe der Formel -NR⁷-CO-NR⁷₂, Alkylcarbonat-Substituenten der Formel -O-CO-OR⁷, Sulfinsäure der Formel -SO-OR⁷ sowie Derivaten davon, Sulfoxide der Formel -SO-R⁷ sowie Derivate davon, Phosphonsäure, -salz, -ester- oder -amid davon, wobei auch R¹ und R² und/oder R² und R³ jeweils unabhängig voneinander gegebenenfalls substituierte anellierte Ringsysteme aus 1 bis 3 Ringen bilden können, die Heteroatomgruppen enthalten können, oder gegebenenfalls substituierte Alkylengruppen bilden können, die durch Heteroatomgruppen unterbrochen sein können, wobei die anellierten Verbindungen auch wie vorstehend für die Reste R¹, R², R³ angegeben substituiert sein können,
wobei in Sauerstoffatome tragenden Resten diese auch durch Schwefelatome ersetzt sein können,
wobei im Mittel 0,05 bis 100% der im Molekül vorliegenden Reste R¹, R², R³ von Wasserstoff verschieden sein können, oder entsprechenden heterocyclischen Verbindungen, in denen mindestens eine Gruppe -CR¹=, -CR²=, -CR³= durch -N= ersetzt ist,
- R⁵: jeweils unabhängig H, gegebenenfalls substituiertes C₁₋₁₂-Alkyl, wobei COOH, COO-Alkali, COO(Erdalkali)_{0,5}, COONH₄, SO₃H, SO₃-Alkali, SO₃(Erdalkali)_{0,5}, SO₃NH₄, NR⁷₂, N⁺R⁷₃, 1-Pyridinio, 4-Pyridyl, 4-(1-Methyl)pyridinium als Substituenten in Betracht kommen, C₆₋₁₂-Aryl, C₇₋₁₃-Alkylaryl, gegebenenfalls substituiertes C₁₋₁₂-Alkanoyl, z.B. Formyl, Acetyl, Chloracetyl, gegebenenfalls substituiertes C₇₋₁₃-Aryloyl, z.B. Benzoyl, Oligoethylenglycol mit 1 bis 6 Sauerstoffatomen, Oligoethylenglycolether mit 1 bis 6 Sauerstoffatomen, Imidazoylinethyl oder ein entsprechender Rest, in dem ein N-Atom durch einen C₁₋₁₂-Alkylrest substituiert und gegebenenfalls positiv geladen ist und eine C-H Gruppe im Ring durch C-(C₁₋₁₂-Alkyl) ersetzt sein kann, (1-C₄₋₆-Lactam)methyl, das am Ring C₁₋₁₂-Alkyl-substituiert sein kann,
- R⁷: jeweils unabhängig H, C₁₋₁₂-Alkyl, C₆₋₁₂-Aryl
sowie Tautomerenstrukturen davon
oder Metallkomplexen der cyclischen Verbindungen oder Komplexen der cyclischen Verbindungen mit Mineralsäuren,
wobei bei kationischen Cyclen als Gegenionen X⁻ Chlorid, Sulfat, Hydrogensulfat, Phosphat, Hydrogenphosphat, Nitrat, BF₄⁻, Methansulfonat vorliegen,
als Lichtabsorber, Materialien für Löcherinjektionsschichten in OLEDs, als lichtemittierende Verbindungen in OLED, Synergisten zur Dispergierung von Pigmenten oder zur optischen Datenspeicherung.

Die Aufgabe wird vorzugsweise gelöst durch Verwendung von cyclischen Verbindungen der allgemeinen Formel (I) 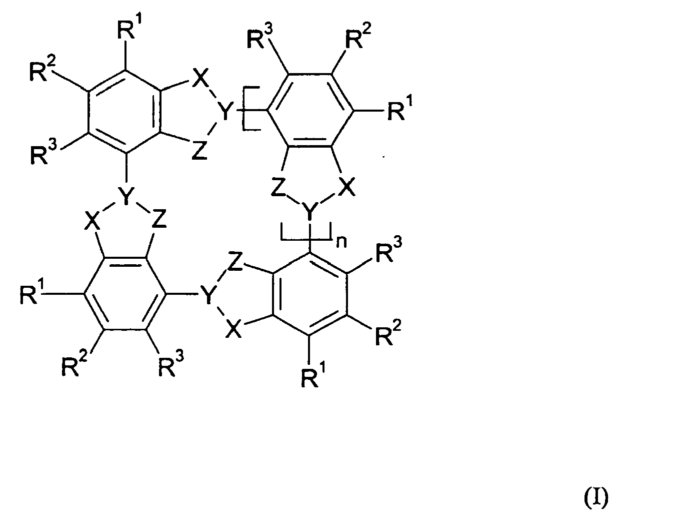 mit der Bedeutung
- n: ganze Zahl im Bereich von 1 bis 7,
- X-Y-Z: jeweils unabhängig O-C=N, N=C-O, NH-C=N, N=C-NH, S-C=N, N=C-S,
- R¹, R², R³: jeweils unabhängig H oder Substituent der Gruppe C₁₋₁₂-Alkyl, C₁₋₁₂-Alkanoyl, C₃₋₇-Cycloalkyl, C₆₋₁₂-Aryl, C₇₋₁₃-Aralkyl, C₇₋₁₃-Alkaryl, C₁₋₁₂-Alkoxy, C₆₋₁₂-Aryloxy, C₁₋₁₂-Hydroxyalkyl, Heterocyclus, C₆₋₁₂-Aroyl, die jeweils substituiert sein können, Hydroxy, Thiol, Halogen, Cyan, Isocyan, Nitro, Ammonium, Amino, Phosphin, Phosphinoxid, Sulfonsäure oder Derivat davon, Carbonsäure oder Derivat davon, Derivate des Siliziums,
wobei auch R¹ und R² und/oder R² und R³ jeweils unabhängig voneinander gegebenenfalls substituierte anellierte Ringsysteme aus 1 bis 3 Ringen bilden können, die Heteroatomgruppen enthalten können, oder gegebenenfalls substituierte Alkylengruppen bilden können, die durch Heteroatomgruppen unterbrochen sein können,
wobei im Mittel 0,01 bis 12 der im Molekül vorliegenden Reste R¹, R², R³ von Wasserstoff verschieden sein können,
oder entsprechenden heterocyclischen Verbindungen, in denen mindestens eine Gruppe -CR¹=, -CR²=, -CR³ durch -N= ersetzt ist,
oder Metallkomplexen der cyclischen Verbindungen,
als Lichtabsorber, Materialien für Löcherinjektionsschichten und Emitter in organischen licht-emittierenden Dioden (OLED), Phasentransferkatalysatoren
oder als Synergisten zur Dispergierung von Pigmenten.
n kann dabei einen Mittelwert darstellen, wenn ein Gemisch von Verbindungen mit unterschiedlicher Anzahl an Ringgliedern vorliegt. Sonst ist n eine ganze Zahl.

Die Aufgabe wird ferner erfindungsgemäß gelöst durch Verwendung von Metallkomplexen der Verbindungen der allgemeinen Formel (I), wie sie vorstehend definiert sind oder als Oxidationskatalysatoren.

Die Verbindungen der allgemeinen Formel (I) zeichnen sich dabei durch eine hohe Stabilität und Langzeitstabilität bei den genannten Anwendungen aus. Durch Wahl eines geeigneten Substitutionsmusters können die Verbindungen der allgemeinen Formel (I) an die unterschiedlichen genannten Anwendungen angepasst werden.

Ein Teil der eingesetzten Verbindungen und Komplexe ist neu. Die Erfindung betrifft deshalb auch cyclische Verbindungen, wie sie vorstehend definiert sind, ausgenommen Verbindungen mit der Bedeutung
- X-Y-Z: jeweils N=C-O, NH-C=N, N=C-NH,
- R¹, R², R³: jeweils H, C₁₋₆-Alkyl.

Die ausgenommenen Verbindungen sind in der eingangs erwähnten Synthesis-Veröffentlichung beschrieben und werden gemäß einer Ausführungsform der Erfindung auch nicht in den erfindungsgemäßen Verwendungen eingesetzt. Dies kann auch speziell für das cyclische Quaterbenzimidazol gelten.

Die Erfindung betrifft auch ein Verfahren zur Herstellung dieser cyclischen Verbindungen der allgemeinen Formel (I) durch Cyclisierung von Verbindungen der allgemeinen Formel (II) 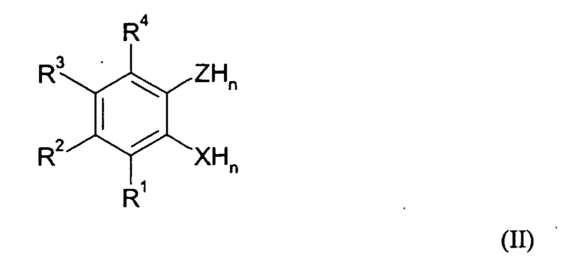 mit der Bedeutung
- R¹, R², R³, X, Z: wie vorstehend für Formel (I) definiert,
- R⁴: -COOH oder Derivat davon,
- n: jeweils 1 oder 2, zum Erhalt der Stöchiometrie,
wobei OH-Gruppen auch als Alkalimetallsalz- oder Ammoniumsalz-Gruppen vorliegen können und/oder NH₂-Gruppen in protonierter Form oder in Derivat-form als -NO, NO₂, -N=N-Aryl, =NOH, =NH vorliegen können, wobei die Cyclisierung in Gegenwart von Metallsalzen, Metallpulvern oder von Lewis-Säuren als Templaten durchgeführt werden kann.

Die Cyclisierung wird z.B. in Gegenwart von Kondensationsmitteln oder unter wasserentziehenden Bedingungen durchgeführt, wobei die Cyclisierung in Gegenwart von Metallsalzen oder Metallpulvern als Templaten oder von Lewis-Säuren durchgeführt werden kann.

Dabei steht R⁴ für Carbonsäure, bzw. deren Derivate wie Carbonsäuresalz, Carbonsäurechlorid, Carbonsäureamid, Carbonsäureester, Carbonsäurenitril, NH₂ steht für unsubstituiertes Amin, sowie Derivate davon wie Ammoniumsalz -NH₃⁺, -NO, -NO₂, Azo -N=N-Aryl, Amid -NCO-Alkyl, -NHOH (Oxim).

Die Herstellung erfolgt beispielsweise unter Erwärmen in gegebenenfalls saurem (H₂SO₄, H₃PO₄, Polyphosphorsäure) Lösungsmittel, gegebenenfalls in Gegenwart von Metallsalztemplaten, sowie gegebenenfalls Oxidations- und Reduktionsmitteln, sowie gegebenenfalls unter Entzug von Amin, gegebenenfalls in Gegenwart eines organischen Lösungsmittels.

Das Verfahren kann einstufig oder auch zweistufig durchgeführt werden, wobei zunächst cyclische Amide/Ester hergestellt und sodann zu cyclischen Heteroaromaten wie Oxazolen weiter umgesetzt werden.

Dabei wird die Cyclisierung vorzugsweise in Gegenwart von Kondensationsmitteln, ausgewählt aus Polyphosphorsäure, (Poly)phosphatestem, Thionylchlorid, Triphenylphosphoniumanhydrid-bis(trifluormethansulfonat) oder unter wasserentziehenden Bedingungen, durchgerührt.

Die vorstehend beschriebenen cyclischen Verbindungen können erfindungsgemäß als Komplexliganden eingesetzt werden. Die Erfindung betrifft auch entsprechende Komplexe, die ein komplexiertes Metallion und mindestens eine cyclische Verbindung, wie sie vorstehend definiert ist, als Komplexliganden enthalten.

Die Erfindung betrifft auch Verfahren zur Herstellung von diesen Komplexen cyclischer Verbindungen durch Herstellung der cyclischen Verbindungen wie beschrieben in Gegenwart von Metallsalzen oder Metallpulvern als Templaten oder durch Umsetzung der cyclischen Verbindungen mit Metallsalzen oder Metallpulvern.

In der vorstehenden Verbindung der allgemeinen Formel (I) bezieht sich der Ausdruck "jeweils unabhängig" auf die einzelnen Positionen im Molekül, in denen die genannten Gruppen auftreten. In jeder der Positionen im Molekül können die Gruppen unabhängig voneinander gleiche oder unterschiedliche Bedeutungen haben. Vorzugsweise zeigt jeder aromatische Kern in den Verbindungen der allgemeinen Formel (I) ein identisches Substitutionsmuster. Sofern X-Y-Z die Bedeutung NH-C=N oder N=C-H hat, ist zu beachten, dass es sich um tautomere Strukturen handelt. N⁺R⁵₂-C=N kann z.B. auch N⁺HR⁵-C=N bedeuten.

Gemäß einer Ausführungsform der Erfindung sind mindestens zwei der aromatischen Kerne unterschiedlich substituiert.

Gemäß einer Ausführungsform der Erfindung haben X-Y-Z und R¹, R², R³ für alle Positionen dieselben Bedeutungen.

In heterocyclischen Systemen sind eine oder mehrere Gruppen -CR¹=, -CR²=, -CR³= durch -N= ersetzt. Vorzugsweise sind in jedem der ringbildenden aromatischen Kerne unabhängig voneinander maximal zwei nicht benachbarte Gruppen durch -N= ersetzt. Jeder der ringbildenden aromatischen Kerne kann auch in identischer Weise -N= im Ring tragen. Es können so Pyridin-, Pyrimidin- oder Pyridazinstrukturen ausgebildet werden. Der Ausdruck -CR¹= beinhaltet beispielsweise ein Kohlenstoffatom des aromatischen Ringes, das zusammen mit dem Substituenten durch -N= ersetzt wird.

Die Reste R¹, R² und R³ können so gewählt werden, dass sie die Cyclisierungsreaktion zur Herstellung der Verbindungen der allgemeinen Formel (I) bzw. (Ia) nicht behindern. Bevorzugte Bedeutungen für die Reste R¹, R² und R³ werden nachstehend detaillierter beschrieben.

X-Y-Z sind vorzugsweise jeweils unabhängig ausgewählt aus O-C=N, N=C-O, NH-C=N, N=C-NH, S-C=N, N=C-S. Vorzugsweise sind die Reste X-Y-Z jeweils unabhängig ausgewählt innerhalb der drei Gruppen O-C=N, N=C-O oder NH-C=N, N=C-NH oder S-C=N, N=C-S. Bei der mittleren Gruppe handelt es sich dabei um tautomere Strukturen. Für die anderen Fälle können insgesamt sechs unterschiedliche Varianten unterschieden werden. Dies soll am Beispiel O-C=N und N=C-O erläutert werden, wobei die Lage des Stickstoffatoms an der Innenseite der cyclischen Struktur oder an der Außenseite der cyclischen Struktur angegeben ist. Es können keine, 1, 2, 3 oder 4 Stickstoffatome innen vorliegen. Im Fall von zwei innen vorliegenden Stickstoffatomen können diese benachbart oder gegenüberliegend sein, so dass sich insgesamt sechs Strukturen ergeben. Gleiches gilt für die Systeme S-C=N, N=C-S.

Die Reste R¹, R², R³ haben vorzugsweise jeweils unabhängig die Bedeutung Wasserstoff oder Substituent der Gruppe
C₁₋₁₂-Alkyl, vorzugsweise C₁₋₆-Alkyl, insbesondere C₁₋₃-Alkyl, die geradkettig oder verzweigt oder cyclisch sein können,
C₆₋₁₂-Aryl, vorzugsweise Phenyl oder Naphthyl,
C₇₋₁₃-Aralkyl, vorzugsweise C₇₋₁₁-Aralkyl, wobei Phenylalkylreste bevorzugt sind, in denen der Alkylrest geradkettig oder verzweigt sein kann,
C₇₋₁₃-Alkaryl, vorzugsweise C₇₋₁₁-Alkaryl, wobei Alkylphenylreste bevorzugt sind, in denen die Alkylgruppe geradkettig oder verzweigt sein kann,
C₁₋₁₂-Alkoxy, vorzugsweise C₁₋₆-Alkoxy, insbesondere C₁₋₃-Alkoxy, wobei der Alkylrest geradkettig oder verzweigt oder cyclisch sein kann,
C₆₋₁₂-Aryloxy, vorzugsweise Phenyloxy oder Naphthyloxy,
C₁₋₁₂-Hydroxyalkyl, vorzugsweise C₁₋₆-Hydroxyalkyl, insbesondere C₁₋₃-Hydroxyalkyl,
wobei die vorstehenden Reste jeweils noch weiter substituiert sein können, beispielsweise durch die nachstehenden Reste,
Hydroxy,
Thiol,
Halogen, vorzugsweise Fluor, Chlor, Brom, besonders bevorzugt Fluor oder Chlor,
Cyan,
Isocyan,
Nitro,
Ammonium,
Amino, das sich von primären, sekundären oder tertiären Aminogruppen ableiten kann, die Alkyl- oder Arylreste aufweisen, die der vorstehenden Definition der Alkylreste und Arylreste entsprechen,
Phosphin oder Phosphinoxid, wobei diese Reste Alkylsubstituenten oder Arylsubstituenten enthalten können, wie sie vorstehend beschrieben sind,
Sulfonsäure oder Derivat davon, wobei die Derivate Säurehalogenide, Säureamide, Säureester sein können,

Carbonsäure oder Derivat davon, wobei die Derivate Säurehalogenide, Säureamide oder Säureester sein können.

Derivate des Siliziums, z.B. Silyl, wobei unterschiedliche Silylgruppen vorliegen können, die Wasserstoffatome, Alkylreste und/oder Alkoxyreste aufweisen können.

Die genannten Alkylreste können durch 1 bis 10 nicht benachbarte Sauerstoffatome unterbrochen sein, so dass sich Etherstrukturen ergeben. R¹ und R² und/oder R² und R³ können auch jeweils unabhängig voneinander gegebenenfalls substituierte anellierte Ringsysteme aus 1 bis 3 Ringen bilden, die Heteroatomgruppen enthalten können. Vorzugsweise enthält jedes derartige anellierte Ringsystem zusätzlich zum in der allgemeinen Formel (I) gezeigten aromatischen Kern 1 bis 2 weitere Ringsysteme. Dabei können an den vier aromatischen Kernen der allgemeinen Formel (I) unterschiedliche anellierte Systeme vorliegen. Die anellierten Ringsysteme können dabei wie vorstehend beschrieben substituiert sein, wobei von Alkylsubstituenten bis zu Silylgruppen alle geeigneten Substituenten vorliegen können. Die anellierten Ringsysteme können zudem Heteroatomgruppen enthalten, so dass Hetarylgruppen gebildet werden. Beispiele geeigneter Grundkörper und anellierter Ringsysteme sind nachfolgend dargestellt.

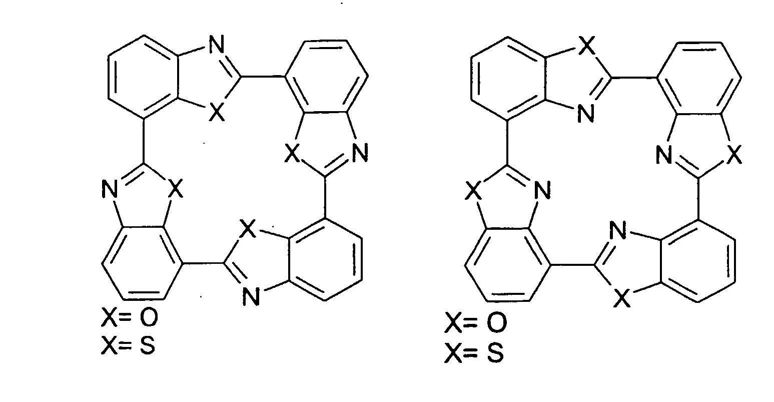

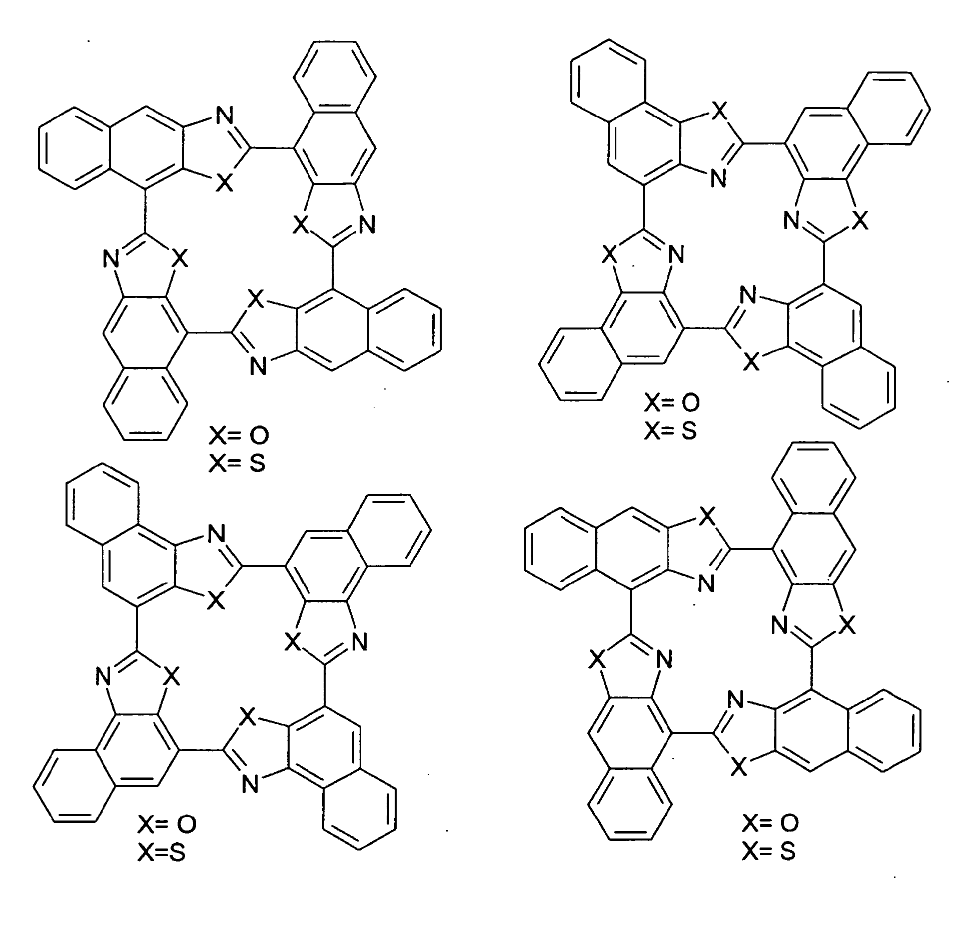

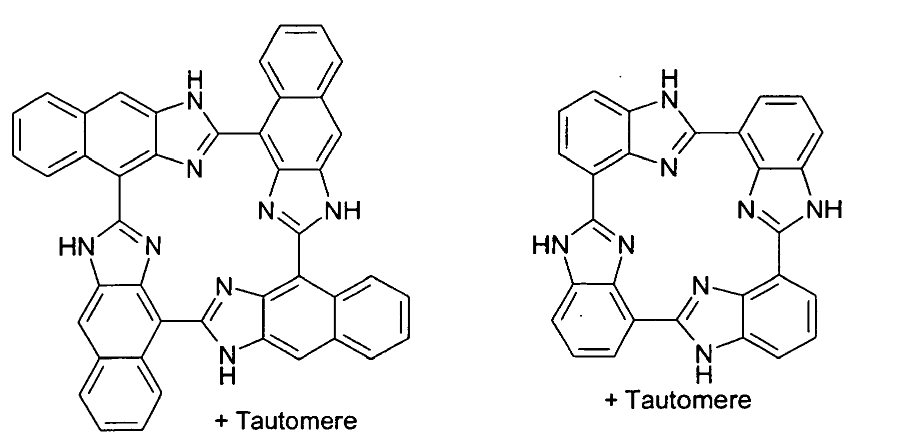

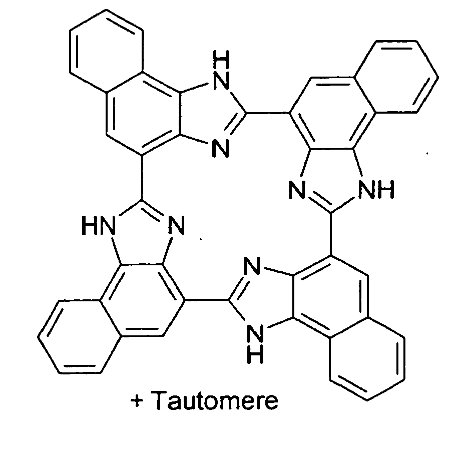

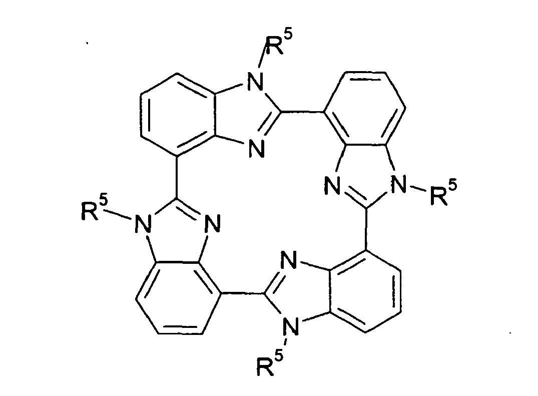

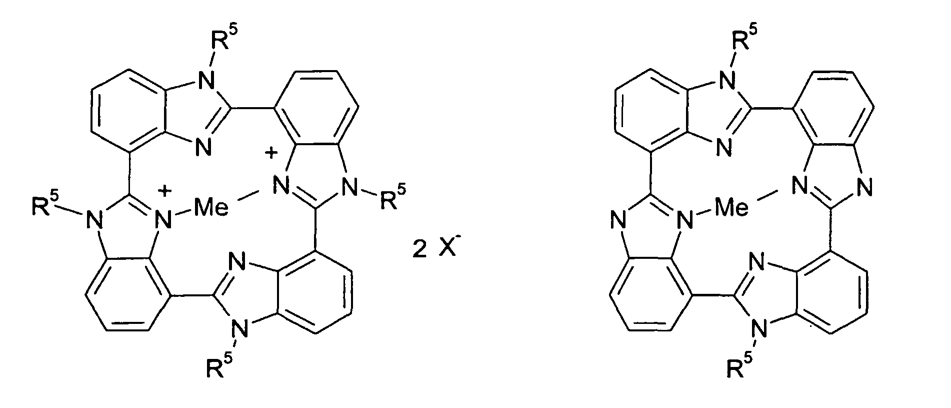

### Me = Metall

Die anellierten Ringsysteme können dabei für jeden der vier aromatischen Kerne der allgemeinen Formel (I) in geeigneter Weise miteinander verknüpft sein. So können beispielsweise linear oder nicht-linear anellierte Strukturen ausgebildet werden.

R¹ und R² und/oder R² und R³ können auch jeweils unabhängig voneinander, gegebenenfalls substituierte, Alkylengruppen bilden, die durch Heteroatomgruppen unterbrochen sein können. Hierdurch entstehen aliphatische oder heteroaliphatische Ringe oder Ringsysteme. Der Abstand zwischen den Anbindungsstellen der Alkylengruppen ist daher vorzugsweise so gewählt, dass sich eine 5-, 6- oder 7-Ringstruktur ergibt. Die Alkylengruppen weisen vorzugsweise 2 bis 10 Kohlenstoffatome, besonders bevorzugt 3 bis 10 Kohlenstoffatome auf, wobei vorzugsweise ein 5-, 6- oder 7-Ring ausgebildet wird.

Die Alkylengruppen können geradkettig oder verzweigt sein. Als Heteroatome kommen Sauerstoff-, Schwefel- und Stickstoffatome (NH) in Betracht.

In einem Molekül der allgemeinen Formel (I) können die Reste R¹, R² und R³ für unterschiedliche Molekülgruppen auch unterschiedliche cyclische Strukturen ausbilden. Beispielsweise können anellierte Strukturen neben aliphatischen Ringstrukturen vorliegen.

Erfindungsgemäß können sämtliche der Reste R¹, R² und R³ Wasserstoffatome sein. In diesem Fall handelt es sich um unsubstituierte Ringsysteme. Sofern Substituenten vorliegen, können im Mittel 0,01 bis 12 der im Molekül vorliegenden Reste R¹, R², R³ von Wasserstoff verschieden sein. Vorzugsweise liegen 1 bis 8 Substituenten im Mittel vor. Bei den niedrigen Substitutionsgraden sind die Verbindungen "ansubstituiert". Dies bedeutet, dass unterstöchiometrische Mengen an Substituenten vorliegen, so dass nur ein Teil der Moleküle substituiert ist. In diesem Fall liegen Gemische aus nicht substituierten und substituierten Verbindungen der allgemeinen Formel (I) vor.

Gemäß einer Ausführungsform der Erfindung kann jeder der vorstehend angegebenen Reste durch die anderen der vorstehend angegebenen Reste substituiert sein. Auch anellierte Ringsysteme können nochmals substituiert sein. Ein anelliertes Ringsystem kann beispielsweise durch Alkylreste, Arylreste, Halogenatome, Aminogruppen usw. substituiert sein.

In den Verbindungen der allgemeinen Formel (I) können damit carbocyclische aromatische, carbocyclische nicht aromatische Gruppen, aromatische Heterocyclen oder nicht aromatische Heterocyclen oder Mischungen davon vorliegen. Anellierte Ringe können linear oder verzweigt sein.

Die Erfindung betrifft auch Verbindungen mit n größer 1, da diese Verbindungen bei der Cyclisierung in PPA als Nebenprodukte entstehen. Angewandte Chemie (Heft 114/8 1480-1483, 2002) berichtet über octacyclische Pyrrole mit IR-absorbierenden Eigenschaften, welche durch Cyclisierung in H₂SO₄ hergestellt werden. Dabei fungiert die Mineralsäure als Templat und begünstigt die Bildung der Cyclo[8]Pyrrole. Ähnliche Cyclisierungsprodukte können auch bei der erfindungsgemäßen Chemie entstehen, welche ebenfalls als photoaktive Effektstoffe, vor allem als im sichtbaren transparente Verbindungen Verwendung finden können. Vorausberechnungen ergeben dabei bei n=2,3, etc Abweichungen von der Planarität.

In den erfindungsgemäßen Komplexen liegt ein komplexiertes Metallion und mindestens eine cyclische Verbindung der allgemeinen Formel (I) als Komplexligand vor. Die komplexierten Metallionen können sich dabei ableiten von Metallen der Hauptgruppen, der Übergangsmetalle und der Seltenen Erden des Periodensystems der Elemente. Folgende Elemente können dabei insbesondere genannt werden:

Li, Na, K, Rb, Cs, Fr, Be, Mg, Ca, Sr, Ba, Ra, Sc, Y, La, Ac, Ce, Pr, Nd, Pm, Sm, Eu, Gd, Tb, Dy, Ho, Er, Tm, Yb, Lu, Th, Pa, U, Np, Pu, Am, Cm, Bk, Cf, Es, Fm, Md, No, Lr, Ti, Zr, Hf, V, Nb, Ta, Cr, Mo, W, Mn, Tc, Re, Fe, Ru, Os, Co, Rh, Ir, Ni, Pd, Pt, Cu, Ag, Au, Zn, Cd, Hg, B, Al, Ga, In, Tl, Si, Ge, Sn, Pb, As, Sb, Bi, Se, Te, Po.

Bevorzugte Metalle für OLED-Anwendungen sind Eu, Tb, Re, Ru, Os, Ir, Pt, Cu, Au, Tl, Pb, Bi.

Bevorzugte Metalle für Oxidationskatalysatoren sind Co, Fe, Mn, Cr, Ru.

Besonders bevorzugt enthalten die erfindungsgemäßen Komplexe eine der cyclischen Verbindungen der allgemeinen Formel (I).

Die Herstellung der erfindungsgemäßen cyclischen Verbindungen der allgemeinen Formel (I) oder der erfindungsgemäß eingesetzten entsprechenden cyclischen Verbindungen erfolgt durch Cyclisierung von Verbindungen der allgemeinen Formel (II) 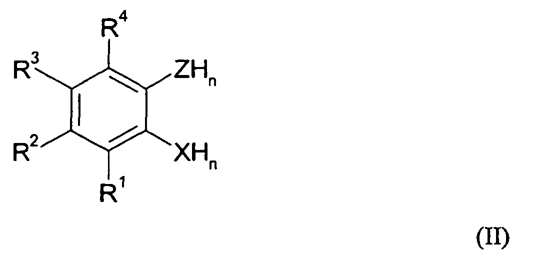 mit der Bedeutung
- R¹, R², R³, X, Z: wie vorstehend angegeben,
- R⁴: -COOH oder Derivat davon,
- n: jeweils 1 oder 2, zum Erhalt der Stöchiometrie,
wobei anstelle von OH-Gruppen auch OAlkalimetall-Gruppen vorliegen können. Die Cyclisierung kann dabei vorzugsweise in Gegenwart von Kondensationsmitteln, ausgewählt aus Polyphosphorsäure, (Poly)phosphatestern, Thionylchlorid, Triphenylphosphoniumanhydrid-bis(trifluormethansulfonat), oder unter wasserentziehenden Bedingungen durchgeführt werden.

Grundsätzlich erfolgt die Herstellung der genannten tetracyclischen Grundkörper aus vier identischen oder unterschiedlichen aromatischen Vorstufen, die die Substituenten R¹, R² und R³ tragen können.

Vorzugsweise stehen in ortho- und meta-Stellung einer Carbonsäure, eines Carbonsäureesters oder eines Carbonsäureamids vorzugsweise Derivate von phenolischen Alkoholen (z.B. O-Silyl) und aromatischen Aminen (z.B. N-carbamat), wobei sowohl das Derivat des phenolischen Alkohols, der unter sauren Bedingungen spaltbar ist, als auch das Derivat des aromatischen Amins orthoständig zum Carbonsäurederivat stehen können.

Die Synthese wird allgemein unter wasserentziehenden Bedingungen, beispielsweise in Gegenwart von Polyphosphorsäure, konzentrierter Schwefelsäure, unter Abdestillierung von Wasser oder Alkohol oder unter Verwendung von Thionylchlorid durchgeführt.

In der vorstehenden Verbindung der allgemeinen Formel (II) ist der Rest R⁴ ein Carbonsäurerest oder ein Derivat davon. Bei dem Derivat handelt es sich vorzugsweise um ein Säurechlorid, einen Ester, ein Amid, ein Salz oder ein anderes entsprechendes Carbonsäurederivat. Ester sind dabei vorzugsweise Ester von niedrigen Alkanolen wie C₁₋₄-Alkanolen. Amide leiten sich dabei vorzugsweise von Ammoniak oder primären Alkylaminen ab.

Wird die Cyclisierung unter Erhitzen in Polyphosphorsäure durchgeführt, wird in der Regel unter Schutzgasatmosphäre (Stickstoff) gearbeitet.

Die Cyclisierung kann lösungsmittelfrei oder in einem Lösungsmittel durchgeführt werden. Geeignete Lösungsmittel sind beispielsweise Sulfolan, Methylenchlorid und Ethylenchlorid. Diese können auch mit Polyphosphorsäure gemischt werden. Die Umsetzung kann bei Raumtemperatur oder erhöhten oder erniedrigten Temperaturen durchgeführt werden. Die Reaktionstemperatur wird in Abhängigkeit von der Reaktivität der jeweils eingesetzten Komponenten gewählt.

Auch der Zusatz von Lewis-Säuren ist möglich, z.B: BF₃ Et₂O usw.. Die Umsetzung kann zudem in Gegenwart von Metallen oder Metallsalzen durchgeführt werden, die als Templat bzw. Katalysator oder als Reduktionsmittel wirken können. Beispielsweise kann die Cyclisierung in Gegenwart von Zinkchlorid oder Kupfersulfat durchgeführt werden. Dabei können auch metallhaltige Cyclen gebildet werden.

Die cyclischen Verbindungen der allgemeinen Formel (I) und deren Metallkomplexe werden erfindungsgemäß als Lichtabsorber oder als lichtemittierende Verbindungen in organischen licht-emittierenden Dioden (OLED) (Leuchtdioden) oder als Oxidationskatalysatoren (nur Metallkomplexe) eingesetzt. Als Lichtabsorber sind sie vorzugsweise UV-Absorber, Vis-Absorber und/oder IR-Absorber. Sie können dabei in einem Anwendungsmedium in löslicher, teillöslicher oder nicht-löslicher Form eingesetzt werden. Der Einsatz als Absorber richtet sich dabei nach der Lage der Absorptionsbande der jeweiligen Verbindung. Die Verbindungen der allgemeinen Formel (I) bilden ein photoaktives System, das Licht der entsprechenden Wellenlänge absorbiert und in Form von Wärme oder Fluoreszenzstrahlung wieder abgibt. Werden die Verbindungen pigmentär eingesetzt, so haben sie vorzugsweise eine mittlere Teilchengröße im Bereich von 5 bis 1.000 nm. Es können Mischungen der Verbindungen als Mischkristalle eingesetzt werden, ebenso Mischungen der Verbindungen mit anderen Farbmitteln/Pigrnenten/Chromophoren wie Phthalocyaninen.

Als UV-Absorber werden sie vorzugsweise als UV-Schutzmittel, die das UV-Licht in Wärme umwandeln, oder als optischer Aufheller, die das UV-Licht in sichtbares Licht umwandeln, eingesetzt. Der Einsatz als UV-Absorber erfolgt beispielsweise in Kosmetika (Sonnenschutz, beispielsweise auch in Kleidung), in Automobildecklacken, in Holzschutzlacken, in Folien, in Kunststoffen (beispielsweise Polystyrol, Polycarbonat, Polyolefine wie auch in ABS- und ASA-Kunststoffen oder PET). Bei den Kunststoffanwendungen können entweder Kunststoff oder auch die Inhalte von Kunststoffbehältnissen geschützt werden. Dies trifft insbesondere für durchsichtige oder durchscheinende Kunststoffflaschen zu. Gleiches gilt auch für aus den Kunststoffen hergestellte Fasern, die beispielsweise zur Herstellung von Kleidung eingesetzt werden (beispielsweise Polyamide). Optische Aufheller werden beispielsweise in Waschmitteln, Textilien oder Kunststoffen eingesetzt. Die Kunststoffe können beispielsweise auch zu Folien weiterverarbeitet werden.

Handelt es sich bei den cyclischen Verbindungen um Vis-Absorber, so können sie als lösliche Farbstoffe oder als unlösliche Pigmente vorliegen. Die löslichen Farbstoffe können die sichtbare Strahlung entweder in Wärme umwandeln oder, als Fluoreszenzfarbstoffe, in Licht. Als Pigmente wandeln sie das Licht insbesondere in Wärme um. Als Buntpigmente finden sie Verwendung zur Färbung von polymeren Materialien wie Farben und Lacken, Kunststoffen, Druckfarben in einer Vielzahl von Anwendungsgebieten, beispielsweise im Automobilbereich.

Sind die Verbindungen in organischen Lösungsmitteln, Polymeren oder Wasser löslich, so können sie als lösliche UV-Absorber, Vis-Absorber (Farbstoffe) in den vorstehenden Anwendungen eingesetzt werden.

Die in Wasser, organischen Lösungsmitteln oder in polymeren Materialien löslichen Verbindungen, insbesondere Sulfonsäuren, Sulfonsäureester, Sulfonsäureamide, die Alkyl- und Arylsubstituierten Vertreter sowie die Metallkomplexe der genannten Verbindungen können je nach Lage des Absorptionsmaximums entweder als UV-absorbierende Substanzen eingesetzt werden, die entweder die absorbierte Energie in Form von Wärme (lösliche UV-Absorber) oder in Form von Licht (optische Aufheller) abgeben. Sie können als Farbstoffe zum Färben von Textilien, Kunststoffen, Papierfasern und als Pigmentdispergieradditive eingesetzt werden.

Lösliche UV-Absorber können beispielsweise in kosmetischen Formulierungen, in Automobildecklacken, in Holzschutzlacken oder in Folien verwendet werden.

Optische Aufheller können zur Aufhellung von Papier, natürlichen Textilfasern, Kunststofffasern in Waschmitteln, zur optischen Aufhellung von Kunststoffen usw. eingesetzt werden.

Die erfindungsgemäßen cyclischen Verbindungen bzw. deren Metallkomplexe, insbesondere Sulfonsäuren, Sulfonsäureester, Sulfonsäureamide usw. können als Dispergieradditive (Synergisten) von allen bekannten (organischen) Pigmenten eingesetzt werden. Als farblose Synergisten können sie mit einer Vielzahl unterschiedlicher Pigmente kombiniert werden, ohne durch eine Eigenfarbigkeit die Farbe zu beeinflussen. Sie können beispielsweise in lösungsmittelhaltigen High Solid-Lacken verwendet werden.

Als pigmentäre anorganische UV-Absorber für Kosmetik, Lack und Kunststoff gibt es nanopartikuläres ZnO (De19907704, EP0449 888). Diese haben jedoch den Nachteil, dass sie bei ungenügender Feinheit weißes Licht streuen, so dass ein milchiges Aussehen hervorgerufen wird. Ungenügende Feinheit tritt bei zu großer Partikelgröße auf, wobei entweder die Primärpartikel zu groß sind oder der Dispergierzustand nicht ausreichend ist. Die beanspruchten Verbindungen weisen ein ähnliches Absorptionsspektrum wie ZnO auf, streuen jedoch als organische Pigmente in den beschriebenen Formulierungen weniger.

Die Erfindung betrifft auch die Verwendung der Lichtabsorber zum Einfärben von hochmolekularen organischen Materialien, z.B. Polymeren und ähnlichen Werkstoffen. Die Erfindung betrifft auch thermoplastische Formmassen, Lacke und Beschichtungszusammensetzungen, die die Lichtabsorber in üblichen Mengen enthalten.

Gemäß einer weiteren Ausführungsform der Erfindung können die erfindungsgemäßen Verbindungen in OLEDs eingesetzt werden. Sie können dabei insbesondere als Emitter in der Ermitterschicht oder für die Löcherinjektionsschicht zwischen Anode und Löcherleiterschicht in Frage kommen und in der letztgenannten Anwendung beispielsweise Kupferphthalocyanin ersetzen. Ein Vorteil gegenüber Kupferphthalocyanin ist eine geringere Absorption im sichtbaren Spektralbereich. Weiterhin können schwermetallhaltige Komplexe als Triplett-Emitter in OLEDs verwendet werden. Für OLED-Anwendungen werden metallfreie und Metallkomplexe der erfindungsgemäßen Verbindungen eingesetzt, wobei vorzugsweise als Metall Cu, Zn und/oder Pt eingesetzt werden.

Die erfindungsgemäßen Verbindungen können zudem in Form von Metallkomplexen als Oxidationskatalysatoren eingesetzt werden. Besonders bevorzugte Oxidationskatalysatoren sind Komplexe der erfindungsgemäßen Verbindungen mit Metallen, ausgewählt aus Co, Fe, Mn, Cr, Ru und Mischungen davon.

Die Herstellung der erfindungsgemäßen Verbindungen wird nachstehend anhand von Syntheseschemata und von Beispielen näher erläutert.

Erläuterungen zur Synthese der unsymmetrischen Cycloquaterverbindungen über Peptidverknüpfungsreaktionen:

Die linearen Peptide bzw. Ester werden aus den Einzelbausteinen der Formel (II) nach Standardreaktionen mit Standardschutzgruppen hergestellt. Beispiele dazu findet man auch in "Amino Acid and Peptide Synthesis", John Jones, Oxford University Press 1992.
Die Cyclisierungen erfolgen nach der Methode von U. Schmidt an Phasengrenzflächen, um hohe Verdünnungen zu vermeiden.
Literatur dazu findet sich in: .
U. Schmidt et al. Synthesis 1992, 1248-1254
U. Schmidt et al. Synthesis 1992, 1025-1030
U. Schmidt et al. Tetrahedron Lett. 1988, 29, 4407-4408
U. Schmidt et al. Synthesis 1987, 236-241
U. Schmidt et al. J. Org. Chem. 1982, 47, 3261-3264
U. Schmidt et al. Angew. Chem. Int. Ed. Engl. 1981, 20, 280-281
CBz steht für Benzyloxycarbonyl
Boc steht für tert-Butoxycarbonyl
TBS steht für ter-Butyldimethylsilyl
Für weitere Schutzgruppen, sowie deren Anbringung und Entfernung siehe: T.W. Greene P.G.M. Wuts, Protective Groups in Organic Synthesis 1991 John Wiley& Sons.
Für weitere Abkürzungen siehe die gleiche Literaturstelle.

Die ersten beiden Schemata betreffen Ringstrukturen, die keine innenliegenden Sauerstoffatome in den Fünfringen aufweisen. Es folgen Schemata für 1, 2, 3 und 4 innenliegende Sauerstoffatome.
Reste 1, Reste 2, Reste 3, Reste 4 beschreiben mögliche Substituenten der Ringsysteme.

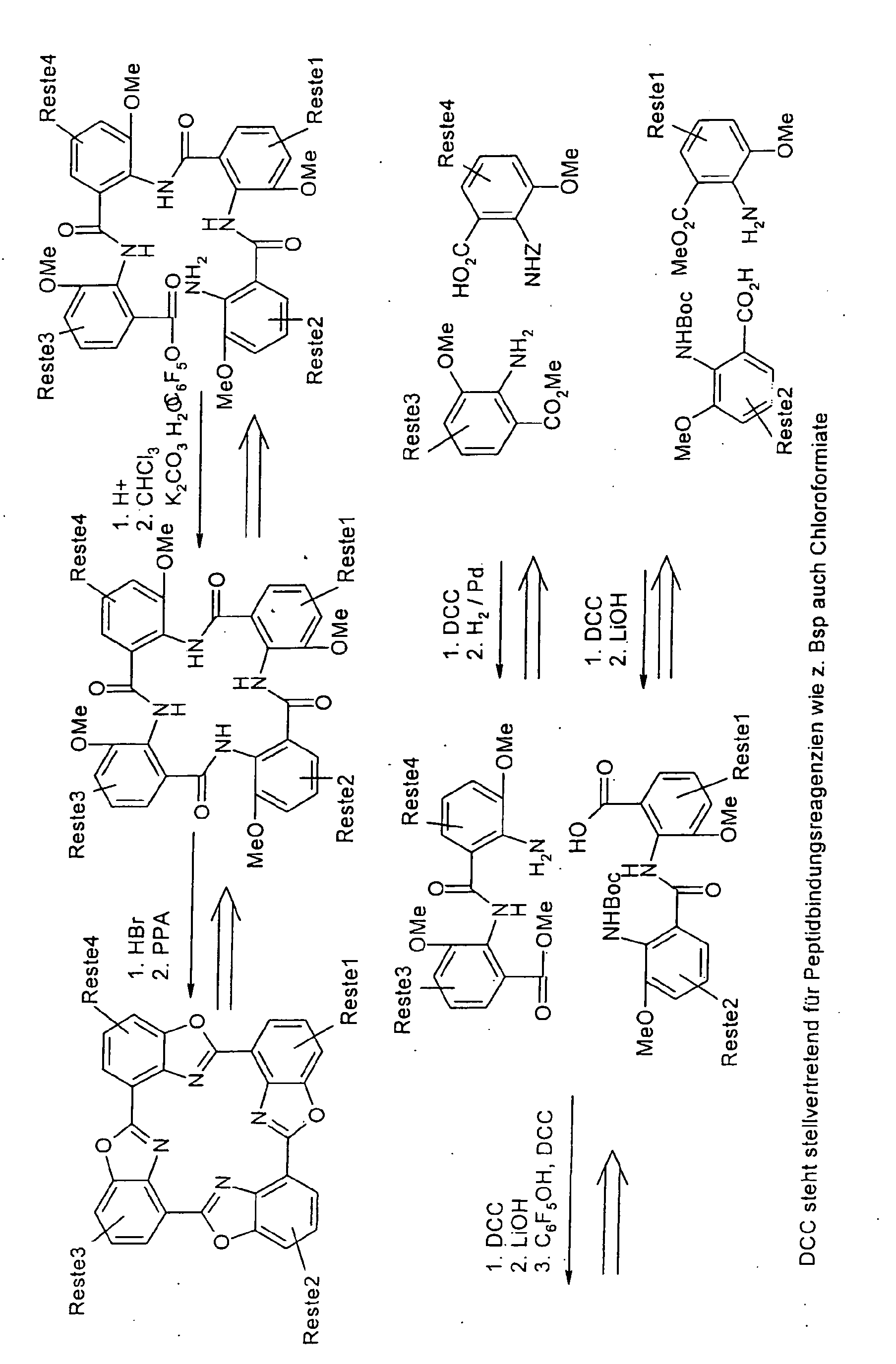

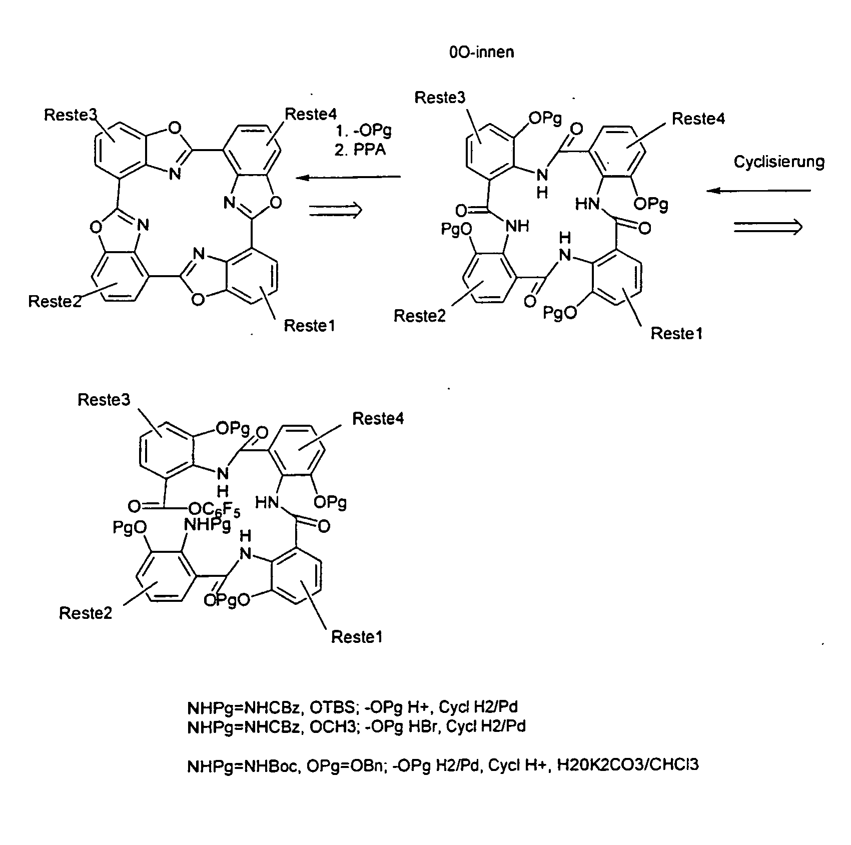

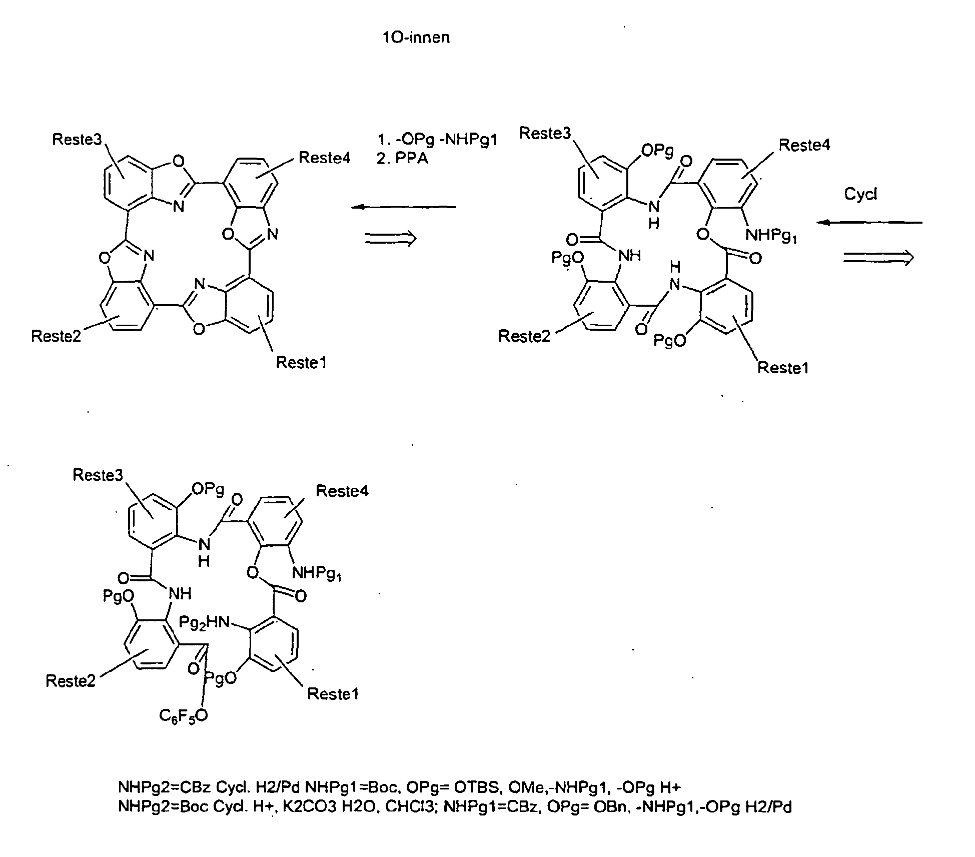

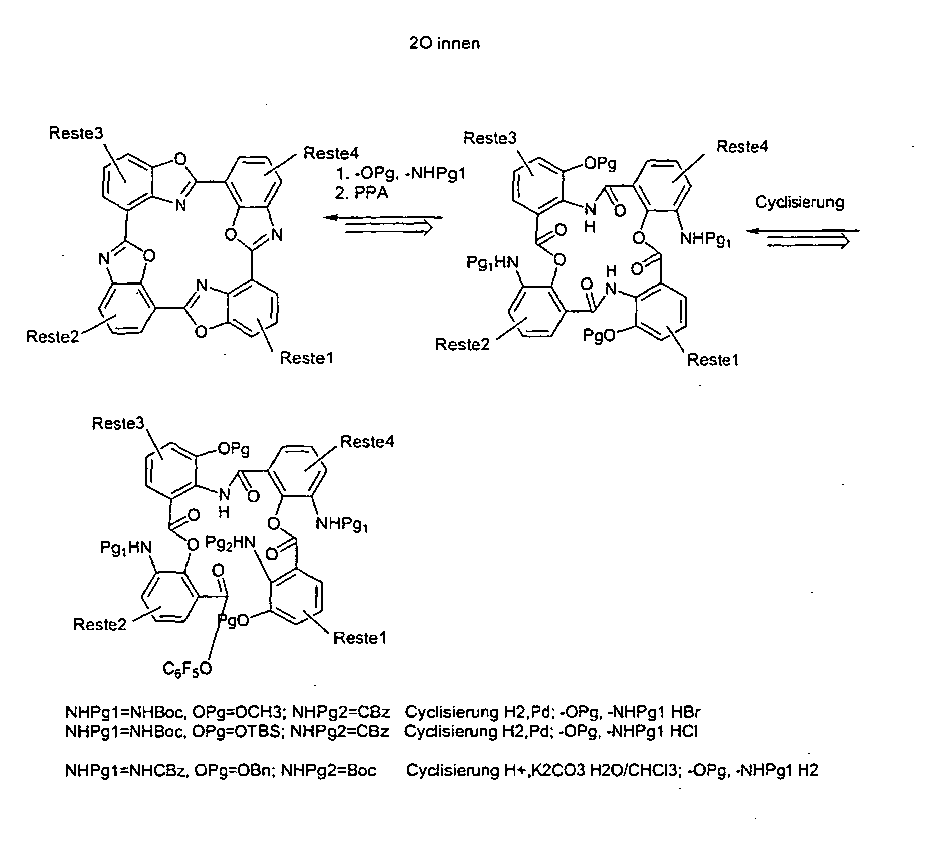

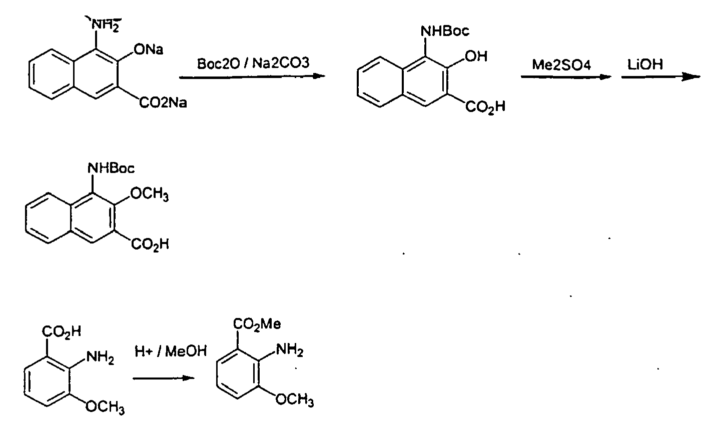

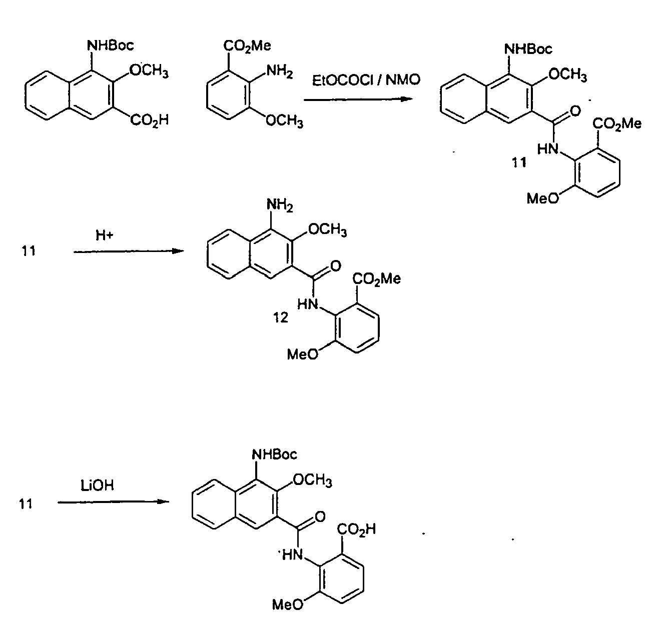

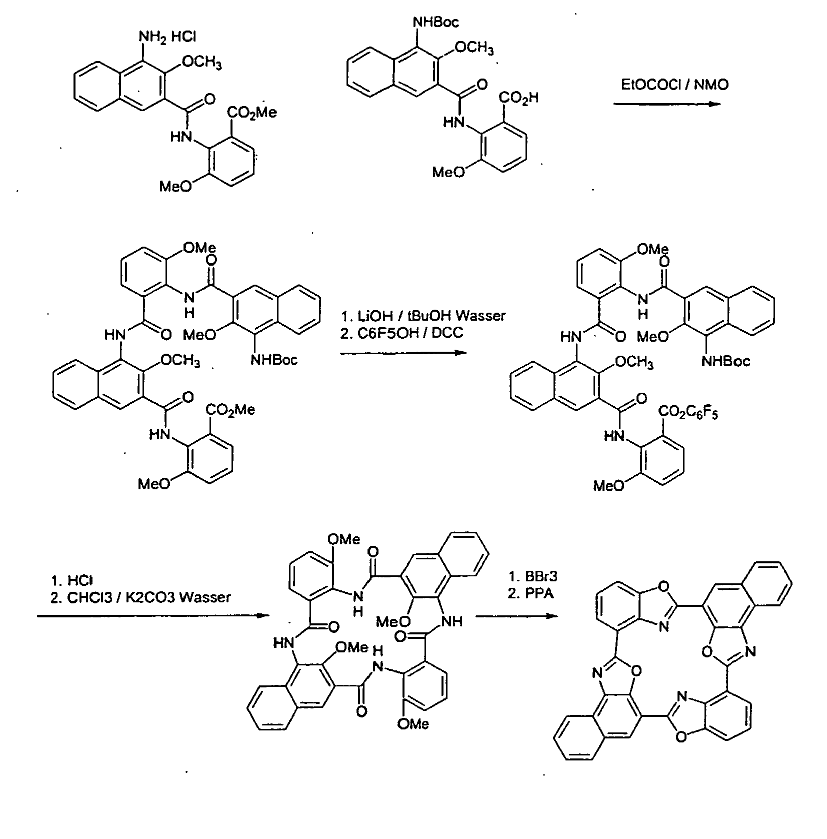

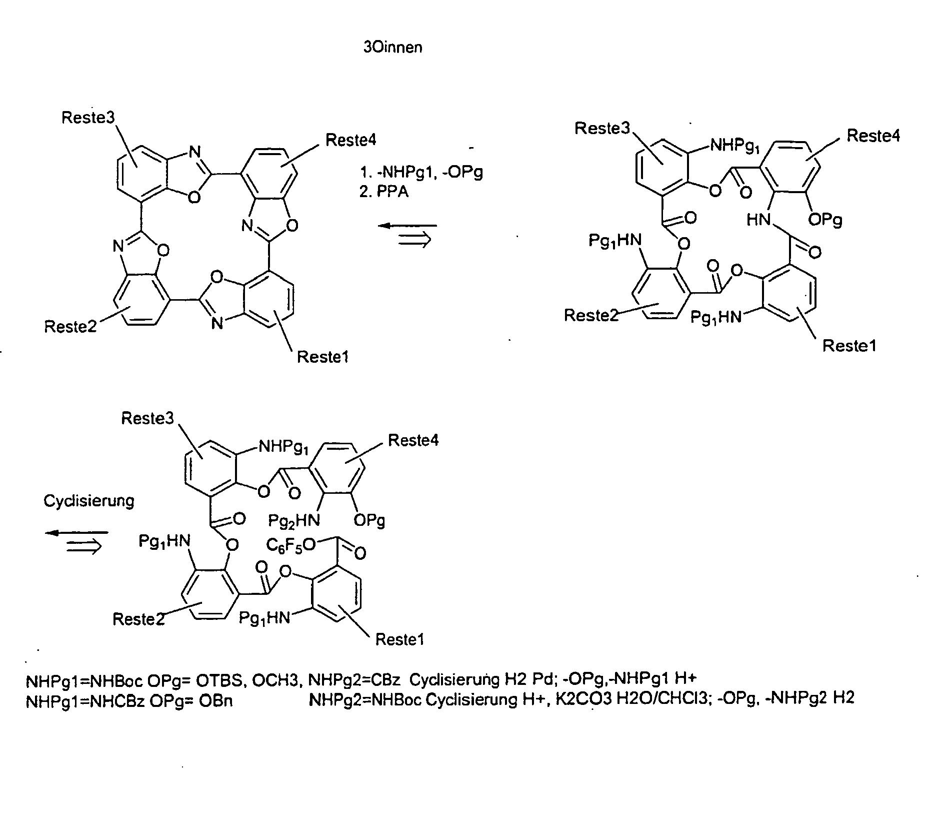

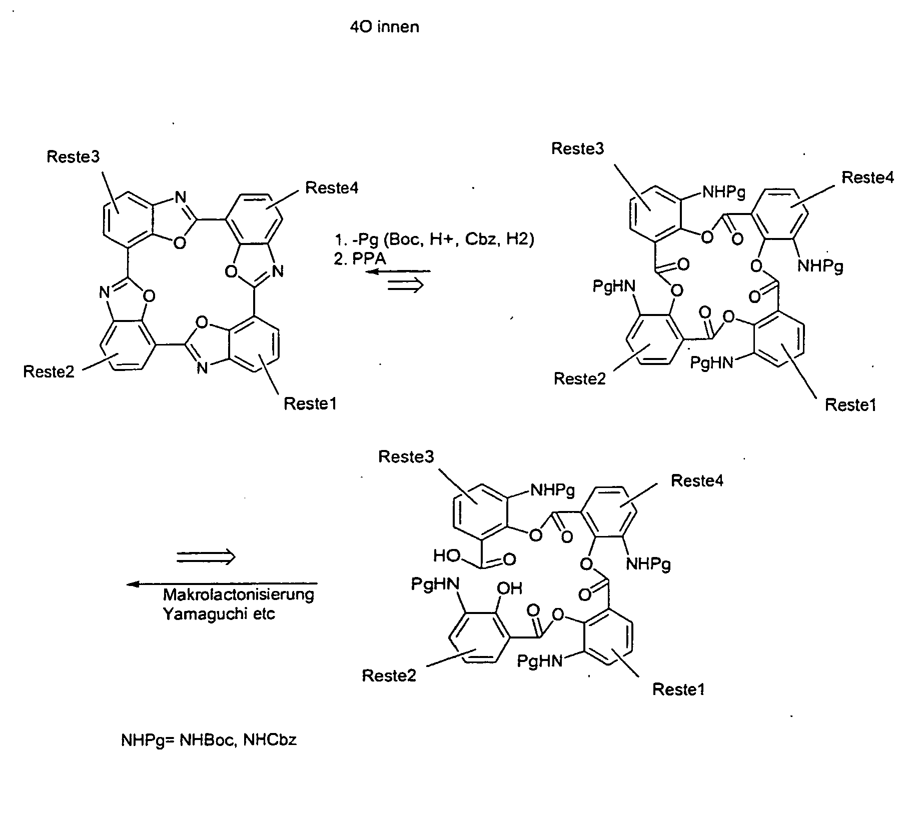

Die Erfindung wird durch die nachstehenden Beispiele näher erläutert:

### Beispiele

### Beispiel 1

### Cyclo-2,4':2',7'':2'',4''':2''',7-quaterbenzimidazol /

### Cyclo-2,4':2',7''(4''):2'',4'''(7'''):2''',7''''(4''''):2 '''',4(7)-quinquebenzimidazol

In 240 g 100 °C heiße 85 %ige Polyphosphorsäure wurden unter Rühren und Stickstoff 20.7 g (122 mmol) Ammonium-2,3-diamino-benzoat portionsweise eingetragen. Anschließend wurde die Reaktionslösung unter Gasentwicklung (Ammoniak) auf 160 °C erhitzt und 24 Stunden bei dieser Temperatur gehalten. Nach Abkühlen der Lösung auf 60 °C wurde diese in 400 ml Eiswasser gegossen, wobei ein Niederschlag ausfiel. Der Niederschlag wurde abgesaugt und mit 200 ml Wasser gewaschen. (Die saure Mutterlauge wurde zum Cycloquinquebenzimidazol aufgearbeitet; siehe weiter unten.) Das feuchte Nutschgut wurde in 500 ml Wasser suspendiert. Die Suspension wurde mit Ammoniaklösung alkalisch gestellt und dann filtriert. Der Feststoff wurde mit Wasser gewaschen und im Vakuum bei 70 °C getrocknet. Es wurden 14.2 g schwarzbraunes Rohprodukt erhalten. Zur Reinigung wurden 14.0 g Rohprodukt in 280 g 96 %ige Schwefelsäure bei 10 - 20 °C innerhalb von 2 Stunden eingetragen. Ein gelblicher Feststoff wurde durch tropfenweises Zugeben von 250 ml Wasser innerhalb von 2 Stunden ausgefällt. Nach Rühren über Nacht bei Raumtemperatur wurde die Suspension über eine Glasfritte filtriert. Der Feststoff wurde mit 200 g 50 %iger Schwefelsäure nachgewaschen. Das Nutschgut wurde in 400 ml Eiswasser suspendiert, abgesaugt, mit Wasser gewaschen und bei 60 °C im Vakuum getrocknet (4.6 g). In einem zweiten Reinigungsschritt wurde dieses einmal durch Fällung gereinigte Produkt in 90 g 96 %ige Schwefelsäure bei 10 - 20 °C gelöst. Nach zweistündigem Rühren wurden 200 g 50 %ige Schwefelsäure innerhalb von 2 Stunden zugetropft. Die Suspension wurde über Nacht gerührt, dann über eine Glasfritte abgesaugt und mit 50 g 60 %iger Schwefelsäure nachgewaschen. Das Nutschgut wurde in 400 ml Wasser suspendiert und mit Natronlauge auf pH 12 versetzt. Der Feststoff wurde abgesaugt, mit Wasser gewaschen und im Ölpumpenvakuum bei 85 °C getrocknet. Ausbeute: 1.32 g (9.3 %) grünlich gelbe Mikrokristalle
Laut Elementaranalyse kristallisierte der Makrocyclus mit einem halben Moläquivalent Wasser.

| | | | |
|---|---|---|---|
| C₂₈H₁₆N₈ x 0.5 H₂O | Ber. C 71.03 | H 3.62 | N 23.67 |
| M = 464.49 + 0.5 x 18.02 = 473.50 | Gef. C 70.8 | H 3.47 | N 23.5 |

MALDI-MS: [M + H]⁺ = 465.2
UV/Vis: λₘₐₓ (lg ε) = 356 (S), 320 (4.82), 264 (4.58), 220 nm (4.33) in konz. Schwefelsäure, λ_{fluor.} = 464 nm in Ameisensäure

Die saure Mutterlauge wurde mit Natronlauge auf pH 8 - 9 gestellt, wobei ein Niederschlag ausfiel, der abgesaugt, mit Wasser gewaschen und im Vakuum bei 70 °C getrocknet wurde. Es wurden 1.8 g brauner Feststoff erhalten, der zweimal aus Ethylenglykol umkristallisiert wurde.
Ausbeute: 1.48 g (8 %) farblose Mikrokristalle
Laut Elementaranalyse und ¹H-NMR in D₆-DMSO kristallisierte der Makrocyclus mit 1.5 Moläquivalenten Wasser.

| | | | |
|---|---|---|---|
| C₃₅H₂₀N₁₀ x 1.5 H₂O | Ber. C 69.18 | H 3.82 | N 23.05 |
| M = 580.61 + 1.5 x 18.02 = 607.63 | Gef. C 69.3 | H 3.47 | N 23.1 |

MALDI-MS: [M + H]⁺ = 581.2
IR (KBr): 3398(s), 3185 (2), 1621, 1537, 1484, 1425 (s), 1368, 1319, 1280, 1238 (s), 1063, 930, 860, 799 (s), 750 (s) cm⁻¹
¹H-NMR (400 MHz, D₆-DMSO): 13.1 (mc; 4H, NH), 12.05 (mc; 1H, NH) 7.8 (mc: 15H, Aromaten-H), 3.4 (s; 3H, H₂O)
UV/Vis: λₘₐₓ (lg ε) = 354 (S), 3.10 (4.82), 256 (4.57), 220 nm (4.43) in konz. Schwefelsäure, λₘₐₓ (lg ε) = 354 (S), 314 nm (4.84) in Ethanol, λ_{fluor}. = 461 nm in Ameisensäure

### Beispiel 2

### Bis(2-amino-3-carboxyphenylammonium)hydrogenphosphat (a)

47.50 g (281 mmol) Ammonium-2,3-diaminobenzoat wurden in einer Lösung aus 675 ml Aceton und 225 ml Wasser unter Rückfluss zum Sieden erhitzt. In der Siedehitze wurden 32.37 g (281 mmol) 85 %ige o-Phosphorsäure unter Rühren innerhalb von 30 min zugetropft. Die Lösung wurde 20 min nachgerührt und heiß filtriert. Das Filtrat wurde bis zur Trockne eingeengt. Nach Trocknen im Vakuum bei 60 °C wurden 73.2 g rötliches Pulver (Schmp. 204/205 °C unter Zers.) erhalten, das aus 1250 ml Wasser umkristallisiert wurde.
Ausbeute: 39.3 g (69.5 %) dunkelrote Kristalle; Schmp. 207 °C (Zers.)

| | | | | |
|---|---|---|---|---|
| C₁₄H₁₉N₄O₈P | Ber. C 41.80 | H 4.76 | N 13.93 | P 7.70 |
| M = 402.30 | Gef. C 41.9 | H 4.7 | N 14.0 | P 7.4 |

### Cyclo-2,4':2',7'':2'',4''':2''',7-quaterbenzinüdazol / Cyclo-2,4':2',7''(4''):2'',4'''(7'''):2''',7''''(4''''):2'''',4(7)-quinquebenzimidazol (b)

In 240 g 100 °C heiße 85 %ige Polyphosphorsäure wurden unter Rühren und Stickstoff 20.92 g (52.0 mmol) Bis(2-amino-3-carboxyphenylarnmonium)hydrogenphosphat portionsweise eingetragen. Anschließend wurde die Reaktionslösung auf 150 °C erhitzt und 24 Stunden bei dieser Temperatur gehalten. Nach Abkühlen der Lösung auf 60 °C wurde diese in 1000 ml Eiswasser gegossen, wobei ein Niederschlag ausfiel. Der Niederschlag wurde abgesaugt und mit 500 ml Wasser gewaschen. (Die saure Mutterlauge wurde zum Cycloquinquebenzimidazol aufgearbeitet; siehe weiter unten.) Das Rohprodukt wurde wie unter 1) beschrieben durch Fällung in Schwefelsäure gereinigt.
Ausbeute: 1.23 g (10 %) grünlich gelbe Mikrokristalle
Die Aufarbeitung der sauren Mutterlauge erfolgte wie unter 1) beschrieben.
Ausbeute: 0.54 g (3.4 %) farblose Mikrokristalle

### Beispiel 3

### 4,12-Dimethyl-cyclo-2,4':2',7'':2'',4''':2''',7-quaterbenzimidazol

Zu einer Suspension von 0.465 g (1.00 mmol) Cycloquaterbenzimidazol und 0.652 g (2.00 mmol) Cäsiumcarbonat in 80 ml wasserfreiem Dimethylformamid wurden 0.340 g (2.40 mmol) Methyliodid unter Rühren bei Raumtemperatur zugetropft. Die Suspension wurde 19 Stunden lang bei Raumtemperatur gerührt. Der Feststoff wurde abgesaugt, mit Dimethylformamid und Wasser gewaschen und bei 60°C im Vakuum getrocknet (0.33 g). Der Feststoff wurde aus 120 ml N-Methyl-2-pyrrolidon umkristallisiert, abgesaugt, mit N-Methyl-2-pyrrolidon, Isopropanol und tert.-Butylmethylether gewaschen, trocken gesaugt. Lösungsmittelreste wurden bei 200°C im Ölpumpenvakuum entfernt.
Ausbeute: 0.205 g (42%) gelbliche Mikrokristalle

| | | | |
|---|---|---|---|
| C₃₀H₂₀N₈ | Ber. C 73.16 | H 4.09 | N 22.75 |
| M = 492.54 | Gef. C 72.9 | H 4.3 | N 22.7 |

MALDI-MS[M + H]⁺: 493.2
¹H-NMR (500 MHz; D₂SO₄): 7.93 (mc; 8H, Aromaten-H), 7.70 (t: 4H, Aromaten-H), 3.97 (s; 6H, N-CH₃)
¹³C-NMR (500 MHz; D₂SO₄): 148.51 (s), 148.21 (s), 136.66 (s), 134.27 (s), 131.98 (d), 131.68 (d), 131.48 (s), 131.26 (d), 131.07 (d), 130.31 (s), 123.39 (d), 121.98 (d), 109.85 (s), 109.33 (s), 34.91 (q)
UV/Vis: λₘₐₓ (1g ε) = 350 (S), 318 (4.84), 262 nm (4.59) in konzentrierter Schwefelsäure λₘₐₓ (1g ε) = 390 (S), 340 nm in Dimethylformamid
IR(KBr): 3384, 1463, 1435, 1415, 1326, 1290, 1281, 1260, 795, 752, 739, 717, 661 cm⁻¹

### Beispiel 4

### 4,8,12,16-Tetramethyl-cyclo-2,4':2',4'':2'',4''':2''',4-quaterbenzimidazol

0.465 g (1.00 mmol) Cycloquaterbenzimidazol werden unter Zugabe von 0.448 g (4.00 mmol) Kalium-tert.-butylat in 50 ml Dimethylformamid bei Raumtemperatur gelöst. Nach Zugabe von 0.848 g (6.00 mmol) Methyliodid wird die Lösung 24 Stunden bei Raumtemperatur gerührt. Der Feststoff wird abgesaugt, mit Dimethylformamid und Wasser gewaschen und bei 60 °C im Vakuum getrocknet.

### Beispiel 5

### 4,8,12,16-Tetramethyl-cyclo-2,4':2',4'':2'',4''':2''',4-quaterbenzimidazol

Zu einer Suspension von 0.465 g (1.00 mmol) Cycloquaterbenzimidazol in 50 ml Dimethylcarbonat wurden bei Raumtemperatur 0.914 g (6.00 mmol) 1,8-Diazabicyclo[5.4.0]undec-7-en getropft. Nach der Zugabe von 1.477 g (4.00 mmol) Tetran-butylammoniumiodid wurde die Reaktionsmischung 91 Stunden lang unter Rühren zum Sieden unter Rückfluß erhitzt. Nach dem Abkühlen wurde der Feststoff abgesaugt, mit Dimethylcarbonat, Aceton und Wasser gewaschen und bei 60 °C im Vakuum getrocknet (0.30 g).

### Beispiel 6

### Kupfer-cyclo-2,4':2',7'':2'',4''':2''',7-quaterbenzimidazol

In 120 g 100 °C heiße 85 %ige Polyphosphorsäure wurden unter Rühren und Stickstoff 4.4 g (26 mmol) Ammonium-2,3-diaminobenzoat und 1.03 g (6.5 mmol) wasserfreies Kupfersulfat eingetragen. Anschließend wurde die Reaktionslösung unter Ammoniakentwicklung auf 160 °C erhitzt und 24 Stunden bei dieser Temperatur gehalten.

Nach Abkühlen der Lösung auf 60 °C wurde diese in 250 ml Eiswasser gegossen, wobei ein Niederschlag ausfiel. Die saure Suspension wurde mit 167 ml konz. Ammoniaklösung neutralisiert und dann filtriert. Der dunkelbraune Rückstand wurde mit Wasser gewaschen und im Vakuum bei 75 °C getrocknet (3.9 g). Das Rohprodukt wurde durch Fällung in Schwefelsäure gereinigt.
Ausbeute: 1.42 g bräunliche Mikrokristalle
SIMS: [M+H]⁺ = 526.1
UV/Vis: λₘₐₓ = 385 (S), 363, 345 nm in Ameisensäure,
λ_{fluor} = 430 nm in Ameisensäure

### Beispiel 7

### Kupfer-cyclo-2,4': 2',7'':2'',4''':2''',7-quaterbenzimidazol

0.465 g (1.0 mmol) Cycloquaterbenzimidazol wurden in 100 ml Dimethylformamid suspendiert und mit 0.200 g (1.0 mmol) wasserfreiem Kupfer(II)-acetat versetzt. Die Reaktionslösung wurde 20 Stunden lang unter Rühren und Stickstoff zum Sieden unter Rückfluss erhitzt. Nach dem Abkühlen der Suspension wurde diese filtriert, der Rückstand mit Dimethylformamid und Wasser gewaschen und bei 60 °C im Vakuum getrocknet.
Ausbeute: 0.223 g bräunliches Pulver

### Beispiel 8

### Nickel-cyclo-2,4':2',7'':2'',4''':2"',7-quaterbenzimidazol

In 120 g 100 °C heiße 85 %ige Polyphosphorsäure wurden unter Rühren und Stickstoff 4.4 g (26 mmol) Ammonium-2,3-diaminobenzoat und 0.842 g (6.5 mmol) wasserfreies Nickelchlorid eingetragen. Anschließend wurde die Reaktionslösung unter Ammoniakentwicklung auf 180 °C erhitzt und 24 Stunden bei dieser Temperatur gehalten. Nach Abkühlen der Lösung auf 60 °C wurde diese in 250 ml Eiswasser gegossen, wobei ein Niederschlag ausfiel. Die saure Suspension wurde mit 175 ml konz. Ammoniaklösung auf pH 8.0 gestellt und dann filtriert. Der dunkelbraune Rückstand wurde mit Wasser gewaschen und im Vakuum bei 75 °C getrocknet (3.30 g). Zur Reinigung wurde das Rohprodukt in 40 ml Ameisensäure bei 80 °C gelöst. Die Lösung wurde filtriert und mit 40 ml n-Propanol versetzt. Nach zweistündigem Rühren bei 80 °C ließ man die Lösung auf Raumtemperatur abkühlen, währenddessen ein Niederschlag ausfiel. Der Feststoff wurde abgetrennt, mit einer Lösung aus Ameisensäure und n-Propanol (1 : 1) nachgewaschen und bei 60 °C im Vakuum getrocknet.
Ausbeute: 0.94 g gelbgrüne Mikrokristalle
SIMS: [M+H]⁺=521.2
UV/Vis: λₘₐₓ = 415 (S), 390 (S), 361 nm in Ameisensäure,
λ_{fluor}. = 470 nm in Ameisensäure

### Beispiel 9

### Magnesium-cyclo-2,4':2',7'':2'',4''':2''',7-quaterbenzimidazol

0.465 g (1.0 mmol) Cycloquaterbenzimidazol wurden in 50 ml Dimethylformamid suspendiert und mit 0.322 g (1.5 mmol) Magensesiumacetat-tetrahydrat versetzt. Die Reaktionslösung wurde 20 Stunden lang unter Rühren und Stickstoff zum Sieden unter Rückfluss erhitzt. Nach dem Abkühlen der Suspension wurde diese filtriert, der Rückstand mit Dimethylformamid und Wasser gewaschen und bei 60 °C im Vakuum getrocknet.
Ausbeute: 0.506 g gelbliche Mikrokristalle
MALDI-MS: [M + H]⁺ = 487.3
UV/Vis: λₘₐₓ = 405 (S), 385, 352 nm in Ameisensäure,
λ_{fluor}. = 492 nm in Ameisensäure

### Beispiel 10

### Platin-cyclo-2,4':2',7'':2'',4''':2''',7-quaterbenzimidazol

2.32 g (5.0 mmol) Cycloquaterbenzimidazol wurden in einer Lösung aus 200 ml Essigsäure und 40 ml Wasser gelöst. Nach der Zugabe von 0.82 g (10 mmol) Natriumacetat wurde die Lösung 10 min lang gerührt und dann mit 2.18 g (5.25 mmol) Kaliumtetrachloroplatinat versetzt. Die Reaktionslösung wurde auf 85 °C erhitzt und 24 Stunden bei dieser Temperatur gehalten. Nach dem Abkühlen auf Raumtemperatur wurde der Feststoff abgesaugt, mit verdünnter Essigsäure und Wasser gewaschen und bei 75 °C im Vakuum getrocknet.
Ausbeute: 3.58 g schwarzbraune Mikrokristalle
MALDI-MS: [M + H]⁺ = 657.1

### Beispiel 11

### Platin-cyclo-2,4':2',7''(4''):2'',4'''(7'''):2''',7''''(4''''):2'''',4(7)-quinquebenzimidazol

0.580 g (1.00 mmol) Cycloquinquebenzimidazol wurden in einer Lösung aus 50 ml Essigsäure und 10 ml Wasser gelöst. Nach der Zugabe von 0.164 g (2.00 mmol) Natriumacetat wurde die Lösung 10 min lang gerührt und dann mit 0.457 g (1.10 mmol) Kaliumtetrachloroplatinat versetzt. Die Reaktionslösung wurde auf 85 °C erhitzt und 44 Stunden bei dieser Temperatur gehalten. Nach dem Abkühlen auf Raumtemperatur wurde der Feststoff abgesaugt, mit verdünnter Essigsäure und Wasser gewaschen und bei 75 °C im Vakuum getrocknet.
Ausbeute: 0.790 g schwarzbraune Mikrokristalle
MALDI-MS: [M + H]⁺ = 773.2

### Beispiel 12

### Cyclo-2,7':2',7'':2'',7''':2''',7-quaterbenzoxazol

In 93.6 g 85 %ige Polyphosphorsäure wurden unter Rühren und Stickstoff 3.90 g (22.9 mmol) Ammonium-3-amino-2-hydroxybenzoat portionsweise eingetragen. Anschließend wurde die Reaktionslösung auf 160 °C erhitzt und 24 Stunden bei dieser Temperatur gehalten. Nach Abkühlen der Lösung auf 85 °C wurde diese in Eiswasser gegossen, wobei ein Niederschlag ausfiel. Der Niederschlag wurde abgesaugt und mit Wasser gewaschen. Das Nutschgut wurde in Wasser suspendiert, und die Lösung mit Ammoniaklösung neutralisiert. Der Feststoff wurde abgesaugt, mit Wasser gewaschen und bei 60 °C im Vakuum getrocknet (1.86 g). Zur Reinigung wurde der Feststoff in konz. Schwefelsäure gelöst und durch langsame Zugabe von Wasser gefällt.
Ausbeute: 0.69 g (25 %) gelbliche Mikrokristalle

| | | | | |
|---|---|---|---|---|
| C₂₈H₁₂N₄O₄ x H₂O | Ber. C 69.14 | H 2.90 | N 11.52 | O 16.45 |
| M = 468.43 + 18.02 = 486.44 | Gef. C 68.4 | H 3.1 | N 11.1 | O 16.4 |

### Beispiel 13

### 2-Acetamido-4-methylphenol(a)

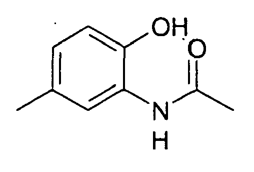

12.3 g (100 mmol) 2-Amino-4-methylphenol wurden in 100 ml absolutem THF gelöst. Zu der braunen Lösung gab man bei Raumtemperatur 10.4 g (102 mmol) Essigsäureanhydrid. Die Temperatur erhöht sich auf 45 °C, und man rührte fünf Stunden ohne weiteres Erwärmen.
Das Lösungsmittel wurde im Vakuum entfernt, und der Feststoff wurde bei 60 °C im Vakuum getrocknet. Die Ausbeute an grauem Pulver ist quantitativ.
¹H-NMR 360 MHz, d6-DMSO δ = 9.50 - 9.45 (b 1H, NH/OH), 9.22 - 9.18 (b, 1H, NH/OH) 7.50 (b, 1H, CH), 6.80 (b, 2H, CH), 2.17 (s, 3H, CH₃), 2.02 (s, 3H, CH₃) ppm.
¹³C-NMR 80MHz, d6-DMSO δ = 168.9 (HNCO), 145.5 (C-O), 126.0, 127.4 (C-CH₃, C-N), 125.0, 122.7, 115.7 (CH, CH, CH), 23.5 (CH₃), 20.3 (CH₃)ppm.
Rf(CHCl₃: Essigsäure=20:5) = 0.6
Smp.: 170-173 °C

### 2-Carboxy-4-methylbenzoxazolidinon(b)

50 g trockenes Kaliumcarbonat wurden in einer Kaffeemühle fein gemahlen, danach wurden 5.0 g (a) (30mmol) innerhalb von 3 mal 20 sec in der Kaffeemühle mit dem Kaliumcarbonat vermahlen. Das weiße Gemenge wurde in einer 250 ml Dose mit 5 Stahlkugeln (Durchmesser 3 cm) auf einem Rollbrett gerollt. Die Kugeln wurden abgetrennt, und das Gemenge wurde für 60 sec in der Kaffeemühle gemahlen, bevor es in einen trockenen Autoklaven eingefüllt wurde. Dieser wurde fünf Minuten evakuiert, bevor 50 bar CO₂ aufgepresst wurden und auf 220 °C aufgeheizt wurden. In der Aufheizphase verbrauchtes CO₂ wurde nachgepresst. Nach Erreichen von 220 °C wurd der Druck auf 100 bar erhöht, und das System wurde 14 Stunden bei dieser Temperatur gehalten. Nach Abkühlen des Autoklaven wurde ein weisser Klumpen entnommen und in einer Kaffeemühle gemahlen. Das Rohprodukt in Gegenwart eines Überschusses von K₂CO₃ wurde folgendermaßen charakterisiert:
¹H-NMR, 360MHz, D₂O δ = 7.18 (s, 1H, CH), 7.02 (s, 1H, CH), 2.28 (s, 3H, CH₃), 1.90 (s, 3H, CH₃) ppm.
¹³C-NMR, 90MHz, D₂O δ = 184.2 (CO), 175.9 (CO), 146.5, 145.9, 135.0, 123.6 (C-OH, C-N, C-CH₃, C-CO₂H), 123.6 (CH), 188.8 (CH), 26.3 (CH₃), 23.3 (CH₃) ppm.
R_{f} (Chloroform:Essigsäure: Methanol=25:5:5)=0.8

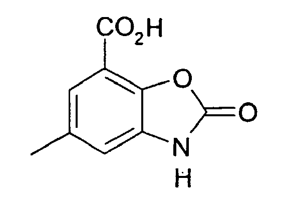

5.0 g (2.7 mmol) der oben beschriebenen Mischung aus K₂CO₃ und Rohprodukt wurden in 5 ml Wasser verrührt, und durch Zugabe von wenig konz. HCl wurde ein pH=1 eingestellt. Die Suspension wurde 44 Stunden bei 50 °C gerührt, und der gebildete Niederschlag wurde nach Abkühlen und Stehen über Nacht abfiltriert. Es wurde mit wenig Eiswasser neutral gewaschen. Nach Trocknen im Vakuum bei 80 °C erhielt man 0.15 g (41 % über drei Stufen) eines weißen Pulvers.
¹H-NMR, 360MHz, d6-DMSO, δ = 11.9 (bs, 1H, NH), 7.30 (s, 1H, CH), 7.03 (s, 1H, CH), 2.02 (s, 3H, CH₃)ppm.
¹³C-NMR, 80MHz, d6-DMSO, δ = 164.7 (CO₂H), 154.5 (NC = OO), 141.0 (C-CO₂H), 132.9, 131.5, 114.1, (C-N, C-O, C-CH₃), 123.0, 114.1 (CH, CH), 20.6 (CH₃)ppm.
R_{f} (Toluol:Ethanol:Essigsäure=25:10:1) = 0.6
Es wurde im Massenspektrum ein Peak mit 193 g/mol (Molekülionenpeak) detektiert.
HRMS (ESI): 192.0296 (gef.)
192.0297 (ber.)
Smp.: 283-286 °C

### Dinatrium-3-amino-5-methylsalicylsäure(c)

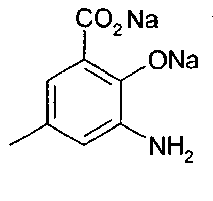

1.01 g (5.23 mmol) (b) wurden in 10 ml Wasser gerührt, und 0.63 g (15.7 mmol) Natriumhydroxid wurden zugegeben. Die rotbraune Lösung wurde 16 Stunden bei Rückfluß gerührt. Danach wurden weitere 0.21 g (5.2 mmol) Natriumhydroxid zugesetzt. Das Wasser wurde bei 80 °C im Vakuum verdampft und man erhielt ein braunes Pulver, welches noch 3eq Natriumhydroxid enthielt.
¹H-NMR, 360MHz, D₂O, δ = 7.15 (s, 1H, CH), 6.90 (s, 1H, CH), 2.17 (s, 3H, CH₃) ppm; die NH₂-Resonanz wurde nicht beobachtet.
¹³C-NMR, 90MHz, D₂O/d6-dmso 1:1 δ = 175.7 (CO₂H), 148.9 (C-OH), 136.8 (C-NH₂(C-CH₃), 128.0 (C-NH₂/C-CH₃), 121.2 (CH), 120.4 (CH), 119.9 (C-CO₂H), 22.3 (CH₃) ppm.
R_{f}(CHCl₃:Methanol:Essigsäure=20:5:1) = 0.7
HRMS (ESI): 166.0504 (gef.)
166.0504 (ber.) entspricht zweifach protoniertem Molekül C₈H₈NO₃

### Tetramethylcycloquaterbenzoxazol (d)

(2,6,10,14-Tetramethyl-cyclo-2,7':2', 7":2", 7"':2"', 7-quaterbenzoxazol)

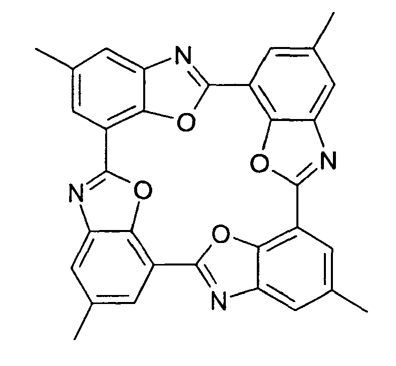

228g Polyphosphorsäure wurden unter Stickstoffatmosphäre auf 180°C erwärmt. 5.7 g (18 mmol) von c, welche noch 2 eq. NaOH enthielt, wurden während 30 Minuten fest zugesetzt. Das Reaktionsgemisch wurde 24 Stunden bei dieser Temperatur gerührt, bevor es in 300 ml 90°C warmes Wasser eingetragen wurde. Zu der grauen Suspension wurden weitere 150 ml Wasser und 200ml n-Butylglykol zugesetzt und 1.5 Stunden auf 100°C erhitzt. Die Suspension wurde heiß abgesaugt. Der Presskuchen wurde mit warmem Wasser neutral gewaschen und anschließend in 100 ml 1M NaOH drei Stunden gekocht. Die Suspension wurde erneut filtriert und mit Wasser und Aceton gewaschen und getrocknet. In 50 ml n-Butylglykol wurde erneut drei Stunden bei 150°C gekocht. Danach wurde filtriert, mit Aceton gewaschen und bei 140°C getrocknet. 1.05 (44%) eines grauen Pulvers wurden erhalten. Dieses wurde in 20g Tetrachlornaphthalin 7 Stunden bei 225°C gerührt, bevor mit 20 ml NMP verdünnt und bei 100°C abfiltriert wurde. Nach Waschen mit Aceton erhielt man 0.91 g (38%) eines hellen Pulvers. Dieses Material wurde in 30g Phenol bei 200°C 5.5 Stunden gerührt, bei 100°C abgesaugt und mit Ethanol, Aceton und Methanol gewaschen. Man erhielt 0.73 g (30%) eines hellgrauen Pulvers. Eine TEM-Aufnahme zeigt ca. 50 nm große Partikel.

C 71.4 (gef.), 73.3 (ber.); H 4.0 (gef.) 3.8 (ber.); N 9.9 (gef.) 10.7 (ber.); O 13.8 (gef.) 12.2 (ber.).

### Beispiel 14

### 5-tert-Octyl-Salicylsäure (a)

80 g (0.39 mol) Natrium wurden in 1200 ml wasserfreiem Xylol geschmolzen und langsam 80g (0.39 mol) tert-Octyl-Phenol eingetragen. Zusätzlich wurden 20 ml THF zugesetzt, um die Rührbarkeit des Gemisches zu verbessern. Nachdem sämtliches Natrium verbraucht wurde, wurden weitere 8.93 g (0.39 mol) Natrium zugesetzt. Unter Rückfluss wurde 20 Stunden CO₂ durch das Reaktionsgemisch geleitet. Durch Zugabe von 250 ml Wasser wurden Reste von nicht umgesetztem Natrium hydrolysiert. Die Phasen wurden getrennt und die organische Phase 3 mal mit je 50 ml 2M NaOH extrahiert. Die vereinigten wässrigen Phasen wurden mit konc. HCl angesäuert, der Niederschlag abfiltriert und neutral gewaschen. Der Niederschlag wurde in 100 ml wässriger NaHCO₃-Lsg und 100 ml Toluol aufgenommen, die Phasen getrennt und die wässrige Phase wiederholt mit je 50 ml Toluol gewaschen. Die wässrige Phase wurde mit konc. HCl angesäuert, der Niederschlag abfiltriert, gewaschen und im Vakuum getrocknet.
47.6 g (48.8%)
¹H-NMR (CDCl₃): δ = 10.4-10.3 (bs, 1H, OH), 7.9 (s, 1H, Ph-H), 7.5 (d, 1H, Ph-H), 6.9 (s, 1H, Ph-H), 1.7 (s, 2H, CH₂), 1.35 (s, 6H, CH₃), 0.7 (s, 9H, CH₃) ppm.

### 5-tert-Octyl-salicylsäuremethylester (b)

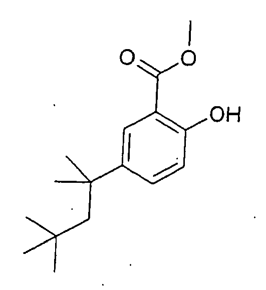

50 ml wasserfreies Methanol und 4.0 g (40.8 mmol) wurden zusammen mit 46.3 g (185 mmol) 3-tert-Octylsalicylsäure 128 Stunden gekocht. Danach wurde das überschüssige Methanol am Rotationsverdampfer entfernt und der Rückstand in einer Mischung aus 100 ml Essigester und 75 ml NaHCO₃ (Vorsicht Schaum) aufgenommen. Die Phasen wurden getrennt und die organische Phase 3 mal mit je 30 ml NaHCO₃ gewaschen. Die organische Phase wurde über MgSO₄ getrocknet und das Lösungsmittel am Rotationsverdampfer entfernt. Man erhält 45.0 g (92%) eines weißen Feststoffes. Rf _{(Toluol : Ethanol = 10:2)} = 0.9
¹H-NMR (CDCl₃) = 10.7-10.6 (bs, 1H, OH), 7.8 (s, 1H, Ph-H), 7.5 (d, 1H, Ph-H), 6.9 (d, 1H, Ph-H), 4.0 (s, 3H , OCH₃), 1.80 (s, 2H, CH₂), 1.25 (s, 6H, CH₃), 0.78 (s, 9H, CH₃) ppm.

### 3-Nitro-5-tert-octyl-salicylsäuremethylester (c)

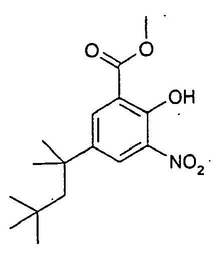

43.62 g (0.165 mmol) des Methylesters (b) wurden zusammen mit 0.1 g Natriumnitrit in 284 g 80%iger H₂SO₄ gelöst und innerhalb von 90 Minuten mit 10.4 g (165 mmol) rauchender Salpetersäure zwischen 5 und 10°C versetzt. Man rührte 6.5 Stunden bei dieser Temperatur, bevor vorsichtig auf Eiswasser gegossen wurde. Die wässrige Phase wurde 3 mal mit je 100 ml Diethylether extrahiert, die vereinigten organischen Phasen wurden mit 50 ml NaHCO₃-Lsg gewaschen, über MgSO4 getrocknet und das Lösungsmittel am Rotationsverdampfer entfernt. Nach Chromatographie (Petrolether / Essigester=9:1) erhielt man 51.1 g (65%) des Produktes. Smp. 60°C.
¹H-NMR (DMSO) δ = 11.2 (bs, 1H, OH), 8.15 (s, 1H, Ph-H), 8.0 (s, 1H, Ph-H), 3.9 (s, 3H, OCH₃), 1.70 (s, 2H, CH₂), 1.25 (s, 6H, CH₃), 0.65 (s, 9H, CH₃) ppm.
¹³C-NMR (DMSO, DEPT) δ = 167.8 (CO₂Me), 150.2 (PhC-OH), 140.8 (PhC-tertOctyl), 138.5 (PhC-NO₂), 131.8 (PhCH), 127.7 (PhCH), 116.3 (PhC-CO₂Me), 55.5 (CH₂), 53.0 (OCH₃), 37.9 (CCH₃), 31.9, 31.5, 30.8 (CH₃) ppm.

### 3-Nitro-5-tert-octyl-salicylsäureamid (d)

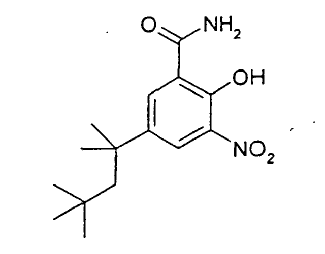

27.45 g (80 mmol) des Esters (c) wurden in 100 ml THF gelöst und 150 ml 1 M NH3-Lösung (150 mmol) zugesetzt. Mit Hilfe einer Gasblase wurde eine NH₃-Atmosphäre von 1bar aufrechterhalten. Man rührte 5 Wochen bei. Raumtemperatur unter diesen Bedingungen, bis ein vollständiger Umsatz erreicht war. Danach wurden die Lösungsmittel im Vakuum entfernt. Der Rückstand wurde mit 300 ml Petrolether gerührt, abfiltriert und im Vakuum getrocknet. Man erhielt 24.8 g (quant.) eines orangen Pulvers.
¹H-NMR (CDCl₃) δ = 12.0-11.0 (b, 1H, OH), 8.7 (s, 1H, Ph-H), 8.25 (s, 1H, Ph-H), 7.9-7.8 (bs, 1H, NH), 6.6-6.5 (bs, 1H, NH), 1.8 (s, 2H, CH₂), 1.4 (s, 6H, CH₃), 0.7 (s, 9H, CH₃) ppm.
¹³C-NMR (CDCl₃) δ = 165.6 (CONH₂), 151.6 (PhC-OH), 142.9 (C-tert.Octyl), 138.7 (PhCH), 134.0 (C-NO₂), 126.2 (PhCH), 121.8 (PhC-CONH₂), 56.3 (CH₂), 38.5 (CH₃), 32.4, 31.9, 31.2 (CH₃) ppm.

### 3-Amino-5-tert-octyl-salicylsäureamid (e)

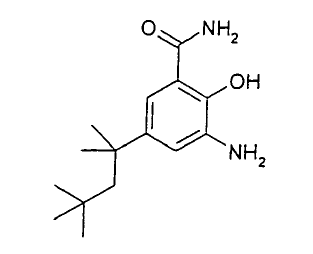

19.7 g (67 mmol) der Nitroverbindung (d) wurden in 100 ml Methanol bei Raumtemperatur gelöst. Man gab 2.0 g Palladium auf Aktivkohle (10%Pd) zu und leitete während 24 Stunden Wasserstoff bei Normaldruck ein. Der Katalysator wurde abgesaugt und das Methanol im Vakuum entfernt. Nach Waschen mit Petrolether, Filtration und Trocknung des Feststoffes erhielt man 15.8 g (89%) eines Feststoffes.
¹H-NMR (DMSO) δ = 8.3 (bs, 1H, CONH₂), 7.8 (bs, 1H, CONH₂), 7.1 (s, 1H, Ph-H), 6.9 (s, 1H, Ph-H), 1.6 (s, 2H, CH₂) 1.2 (s, 6H, CH₃), 0.8 (s, 9H, CH₃) ppm. OH und NH₂ nicht beobachtet.

### Tetra-tert-octylcycloquaterbenzoxazol (f)

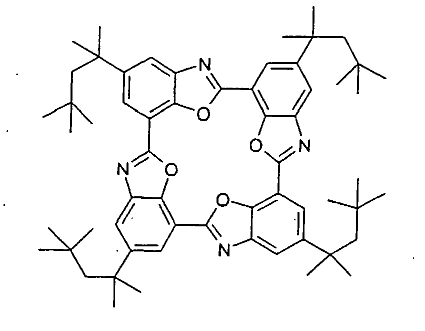

15 g Polyphosphorsäure wurden auf 180°C erwärmt. 200 mg (0.8 mmol) der Verbindung (e) wurden zugesetzt und 24 Stunden bei dieser Temperatur gerührt. Danach wurde die Lösung auf Eiswasser gegeben und der entstandene Niederschlag abfiltriert. Man erhielt 120 mg eines schwarzen Feststoffes. Massenspektroskopisch (MALDI-TOF-SIMS) konnte der Molekülionenpeak bei 917.3 Dalton nachgewiesen werden.

### Beispiel 15

### Cycloquaterbenzoxazol

### (Cyclo-2,4':2',4":2", 4''':2''',4-quaterbenzoxazol)

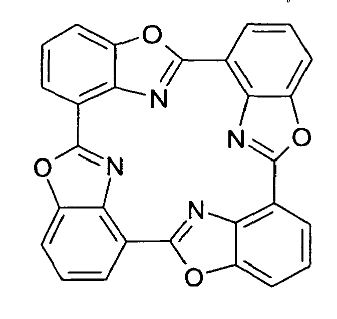

2.00 g (13 mmol) 3-Hydroxyanthranilsäure (Aldrich) werden langsam in 100 ml Polyphosphorsäure bei 180 °C eingetragen. Nach 18 Stunden wird die Lösung auf 250 ml 80 °C warmes Wasser gegeben. Der Niederschlag wird abgesaugt und mit warmen Wasser neutral gewaschen. Man erhält 980 mg 65 % eines ockerfarbenen Niederschlags, welcher bis 350 °C unzersetzt fest bleibt.
Im Massenspektrum wird der Molekülionenpeak des Cyclotetramers mit 469,09 g/mol detektiert, sowie der Molekülionenpeak des Cyclopentamers mit 586,40 g/mol detektiert.
UV-Spektrum aus H₂SO₄λₘₐₓ=262 nm (52 l/cm*g), λₘₐₓ=338 nm (93 l/cm*g).

### Beispiel 16

### 3,4-Diamino-2-naphthoesäure (a)

Azokupplung: 8.74 g (50.0 mmol) 99%ige Sulfanilsäure wurden durch Zugabe von 3.4 g 50%iger Natronlauge in 100 ml Wasser unter Rühren gelöst. Nach der Zugabe einer Lösung von 3.46 g (50.0 mmol) Natriumnitrit in Wasser wurde die Reaktionslösung filtriert und dann langsam zu einer Lösung aus 50 g Eis, 50 ml Wasser und 14.7 g konzentrierter Salzsäure dosiert. Nach 2-stündigem Rühren bei 0 bis 5°C wurde der Nitritüberschuss mit 2 g Amidosulfonsäure vernichtet. Die so erhaltene Diazoniumsalzlösung wurde zu einer Lösung von 11.0 g (50.0 mmol) 85%iger 3-Amino-2-naphthoesäure und 17.85 g (220 mmol) Natriumcarbonat in 500 ml Wasser dosiert, wobei durch Zugabe von 3 g 50% NaOH der pH-Wert oberhalb von 8 gehalten wurde. Die Azofarbstofflösung wurde eine Stunde lang nachgerührt.
Reduktion: Die Lösung des Azofarbstoffes wurde mit 2.86 g 50% NaOH unter rühren versetzt (pH 8.9) und unter Stickstoff auf 80 bis 90°C erhitzt. 23.62 g (136 mmol) Natriumdithionit wurden portionsweise zugegeben, wobei der pH-Wert der Lösung durch Zugabe von 50% NaOH bei ca. 9 gehalten wurde. Nach Entfärben des Reaktionsansatzes wurde dieser auf Raumtemperatur

### Cyclo-2,4':2',9'':2'',4''':2''',9-quaternaphtho[1,2-d]imidazol/ Cyclo-2,4':2',9'':2'',4''':2''',9'''':2'''',4-quinquenaphtho[1,2-d]imidazol (b)

5.00 g (24.9 mmol) 3,4-Diamino-2-naphtoesäure wurden in 120 g 85 %ige Polyphosphorsäure unter Rühren und Stickstoff gelöst. Die Reaktionslösung wurde auf 150 °C erhitzt und 24 Stunden bei dieser Temperatur gehalten. Nach Abkühlen der Lösung auf 60 °C wurde diese in Eiswasser gegossen, wobei ein Niederschlag ausfiel. Der Niederschlag wurde abgesaugt und mit Wasser gewaschen. Das feuchte Nutschgut wurde in heißem Wasser aufgerührt. Die Suspension wurde mit Ammoniaklösung alkalisch gestellt (pH 12), über Nacht gerührt, mit HCl auf pH 8 - 9 gestellt und filtriert. Der Feststoff wurde mit Wasser gewaschen und im Vakuum bei 70 °C getrocknet. Es wurden 3.73 g braunes Rohprodukt erhalten. Zur Reinigung wurde das Rohprodukt in 15.7 g N-Methylpyrrolidon (NMP) suspendiert auf 110 °C erhitzt, wobei ein Teil des Feststoffs in Lösung ging. Die Suspension wurde auf Raumtemperatur abgekühlt und über ein Blaubandfilter filtriert. Der Rückstand wurde viermal mit jeweils 5 ml NMP gewaschen. (Das Filtrat wurde zu Cycloquinquenaphthimidazol aufgearbeitet (siehe weiter unten).) Das Nutschgut wurde in 50 ml Methanol suspendiert, filtriert, mit Methanol gewaschen und bei 40 °C im Vakuum getrocknet (0.90 g). Der gelbliche Feststoff wurde nochmals in 42.7 g NMP suspendiert. Die Suspension wurde bis zum Sieden erhitzt, auf 80 °C abgekühlt und über ein Blaubandfilter filtriert. Der Rückstand wurde mit 6 ml NMP und Ethanol gewaschen und bei 80 °C im Vakuum getrocknet.
Ausbeute: 0.82 g (19 %) gelbliche Mikrokristalle

| | | | |
|---|---|---|---|
| C₄₄H₂₄N₈ x 1.5 H₂O | Ber. C 76.40 | H 3.93 | N 16.20 |
| M = 664.73 + 1.5 x 18.02 = 691.75 | Gef. C 76.8 | H 4.2 | N 16.2 |

MALDI-MS: [M + H]⁺ = 665.2

Das NMP-haltige Filtrat wurde bei 140 °C im Wasserstrahlvakuum zu einer Paste eingeengt, in 160 ml Methanol aufgenommen und 4 Stunden zum Sieden unter Rückfluss erhitzt. Die Suspension wurde auf 40 °C gekühlt, filtriert, viermal mit jeweils 16 ml Methanol gewaschen und bei 80 °C im Umlufttrockenschrank getrocknet (1.68 g). Zur Reinigung wurde der Feststoff in 58.7 g 95.4 %ige Schwefelsäure innerhalb von 30 min eingetragen und war nach 3stündigem Rühren bei 25 °C gelöst. Zu dieser Lösung wurden 53.7 g 50 %ige Schwefelsäure innerhalb von 7.5 Stunden dosiert, wobei sich ein Niederschlag bildete, der über eine G4-Glasfritte abgesaugt und viermal mit insgesamt 23 ml 50 %iger Schwefelsäure gewaschen wurde. Das Nutschgut wurde in 110 ml Wasser suspendiert und eine Stunde lang gerührt. Der Feststoff wurde abgesaugt, mit heißem Wasser gewaschen und bei 80 °C im Umlufttrockenschrank getrocknet (1.10 g). Der Feststoff wurde in 85 ml Ethanol eine Stunde lang unter Rückfluss zum Sieden erhitzt und heiß filtriert. Das Filtrat wurde zur Trockne eingeengt.
Ausbeute: 0.034 g (0.5 %) gelbliches Pulver

Laut Elementaranalyse kristallisierte der Makrocyclus mit vier Moläquivalenten Ethanol.

| | | | |
|---|---|---|---|
| C₅₅H₃₀N₁₀ x 4 C₂H₅OH | Ber. C 74.54 | H 5.36 | N 13.80 |
| M =830.91+4x46.07 =1015.18 | Gef. C 74.5 | H 5.1 | N 13.3 |
| MALDI-MS: [M + H]⁺ = 831.3 | | | |

### Beispiel 17

### Cyclo-2,9':2',9'':2'',9''':2 ''',9-quaternaphtho[1,2-d]oxazol

In 372 g 85 %ige Polyphosphorsäure wurden unter Rühren und Stickstoff 15.5 g (76.3 mmol) 4-Amino-3-hydroxy-2-naphthoesäure portionsweise eingetragen. Die Reaktionslösung wurde auf 160 °C erhitzt und 24 Stunden bei dieser Temperatur gehalten. Nach Abkühlen der Lösung auf 85 °C wurde diese unter Rühren in Eiswasser gegossen, wobei ein Niederschlag ausfiel. Der Niederschlag wurde abgesaugt und solange mit Wasser gewaschen, bis das Waschwasser pH-neutral reagierte. Das Rohprodukt wurde bei 60 °C im Vakuum getrocknet (10.4 g). Zur Reinigung wurde das Rohprodukt in 375 g 95.4 %ige Schwefelsäure gelöst. Die Lösung wurde über eine Glasfritte filtriert und innerhalb von vier Stunden mit 111 g 50 %iger Schwefelsäure versetzt, wobei sich ein Kristallisat bildete. Der Feststoff wurde über eine G4-Glasfritte abgesaugt und jeweils viermal mit 85 %iger und 50 %iger Schwefelsäure gewaschen. Der Rückstand wurde in heißem Wasser aufgerührt. Die Suspension wurde über ein Blaubandfilter filtriert. Der Rückstand wurde mit heißem Wasser pH-neutral gewaschen und bei 80 °C im Umlufttrockenschrank getrocknet.
Ausbeute: 4.56 g (36 %) gelbliche Mikrokristalle; Zers. > 620 °C

| | | | | |
|---|---|---|---|---|
| C₄₄H₂₀N₈O₄ | Ber. C 79.04 | H 3.01 | N 8.38 | O 9.57 |
| M = 668.67 | Gef. C 79.1 | H 3.1 | N 8.4 | O 9.6 |

MALDI-MS: M⁺ = 667.9
UV/Vis: λₘₐₓ (lg ε) = 420 (S), 404 (4.96), 326 (4.69), 268 (4.89), 220 nm (4.71) in
Schwefelsäure, λ_{fluor.} = 484 nm in Schwefelsäure

### Beispiel 18

### 4-Amino-3-hydroxy-2-naphthoesäure

Zu einer Lösung von 10.61 g (25.0 mmol) Calcium-3-hydroxy-2-naphthoat-1-azo-2'-(5'-methyl-benzolsulfonat) (Lithol Rubin Rot) in 500 ml Wasser und 50 g 50 % NaOH wurden bei 80 °C eine Lösung von 35.00 g (200 mmol) Natriumdithionit in 200 ml Wasser zugetropft, wobei der pH-Wert auf 12.0 sank. Die Reaktionslösung wurde zwei Stunden lang zum Sieden unter Rückfluss erhitzt, anschließend auf 60 °C gekühlt und filtriert. Nachdem das Filtrat mit konz. HCl auf pH 4.5 gestellt worden war, wurde der Feststoff abgesaugt, mit Wasser gewaschen und im Exsikkator über Natriumhydroxid getrocknet.
Ausbeute: 3.14 g (< 62 %) gelbliches Pulver; Schmp. 231 - 234 °C (Lit.: 241 °C (Zers.))

### Beispiel 19

### Sulfonierung von Cyclo-2,9':2',9'':2'',9''':2''',9-quaternaphtho[1,2-d]oxazol

5.00 g (7.5 mmol) Cyclo-2,9':2',9'':2'',9''':2''',9-quatemaphtho[1,2-d]oxazol wurden unter Rühren in 75 g 6 %igem Oleum gelöst. Die Lösung wurde auf 150 °C erhitzt und zwei Stunden lang bei dieser Temperatur gerührt. Anschließend wurde die Reaktionslösung auf Raumtemperatur abgekühlt und auf wenig Eis gefällt. Die Suspension wurde mit 2 1 Aceton verdünnt, wobei sich ein feinkristalliner Niederschlag bildete. Der Niederschlag wurde über eine Glasfritte abgesaugt. Das Nutschgut wurde in Aceton suspendiert, abgesaugt, mit Aceton gewaschen und bei 60 °C im Vakuum getrocknet.
Ausbeute: 6.8 g gelbe Mikrokristalle

Laut Elementaranalyse und Massenspektrum lag im Wesentlichen ein vierfach sulfoniertes
Produkt vor.

| | | | | | |
|---|---|---|---|---|---|
| C₄₄H₂₀N₄O₁₆S₄ x 10 H₂0 | Ber. C 45.20 | H 3.45 | N 4.79 | O 35.58 | S 10.97 |
| M = 988.90 + 10 x 18.02 = 1169.05 | Gef. C 46.4 | H 3.8 | N 4.7 | O 35.4 | S 10.3 |

MS (ESI): [M - 4H]⁴⁻ = 245.99, [M - 3H]³⁻ = 328.32, [M - 2H]²⁻ = 492.98

### Beispiel 20

### Sulfonierung von Cyclo-2,9':2',9'':2'',9''':2''',9-quaternaphtho[1,2-d]oxazol

2.50 g (3.75 mmol) Cydo-2,9':2',9'':2'',9''':2''',9-quatemaphtho[1,2-d]oxazol wurden unter Rühren in 37.5 g 6 %igem Oleum gelöst. Die Lösung wurde auf 60 °C erhitzt und 16 Stunden lang bei dieser Temperatur gerührt. Anschließend wurde die Reaktionslösung auf Raumtemperatur abgekühlt und auf 60 g Eis gefällt. Die Suspension wurde mit Aceton verdünnt, wobei sich ein feinkristalliner Niederschlag bildete. Der Niederschlag wurde über eine Glasfritte abgesaugt. Das Nutschgut wurde in Aceton suspendiert, abgesaugt, mit Aceton gewaschen und bei 60 °C im Vakuum getrocknet.
Ausbeute: 1.95 g gelbe Mikrokristalle
Aus der Elementaranalyse für Stickstoff (5.6 %) und Schwefel (9.6 %) wurde ein Sulfonierungsgrad von drei berechnet.

### Beispiel 21

### Sulfonierung von Cyclo-2,9':2',9'':2'',9''':2''',9-quaternaphtho[1,2-d]oxazol

2.50 g (3.75 mmol) Cyclo-2,9':2',9'':2'',9''':2''',9-quaternaphtho[1,2-d]oxazol wurden unter Rühren in 37.5 g 6 %igem Oleum gelöst. Die Lösung wurde auf 60 °C erhitzt und zwei Stunden lang bei dieser Temperatur gerührt. Anschließend wurde die Reaktionslösung auf Raumtemperatur abgekühlt und auf 60 g Eis gefällt. Die Suspension wurde mit Aceton verdünnt, wobei sich ein feinkristalliner Niederschlag bildete. Der Niederschlag wurde über eine Glasfritte abgesaugt. Das Nutschgut wurde in Aceton suspendiert, abgesaugt, mit Aceton gewaschen und bei 60 °C im Vakuum getrocknet.
Aus der Elementaranalyse für Stickstoff (6.3%) und Schwefel (4.8%) wurde ein Sulfonierungsgrad von 1.3 berechnet.

### Beispiel 22

### Chlorierung von Cyclo-2,9':2',9'':2'',9''':2''',9-quaternaphtho[1,2-d]oxazol

30 g Aluminiumchlorid, 6.76 g Natriumchlorid und 0.22 g Natriumbromid wurden zusammen auf 160 °C erhitzt. Die sich dabei bildende Schmelze wurde auf 100 °C abgekühlt und unter Einleitung von Chlor mit 6.68 g (10.0 mmol) Cyclo-2,9':2',9'':2'',9''':2''',9-quaternaphtho[1,2-d]oxazol portionsweise versetzt. Nach Zugabe des Makrocyclus wurde die Temperatur der Reaktionsmischung auf 140 - 150 °C erhöht, wobei fünf Stunden lang Chlor (insgesamt 18.7 g) eingeleitet wurde. Anschließend wurde die Schmelze auf 180 °C aufgeheizt und 30 min bei 180 °C gehalten. Die Schmelze wurde auf Wasser gefällt. Der sich dabei bildende Feststoff wurde abgesaugt, mit Wasser pH-neutral und salzfrei gewaschen und bei 60 °C im Vakuum getrocknet (10.0 g). Zur Reinigung wurde das Rohprodukt in 250 g 97 %iger Schwefelsäure bei 50 - 55 °C gelöst. Bei 40 - 45 °C wurden 86 g 50 %ige Schwefelsäure zugetropft. Nach Abkühlen der Suspension auf Raumtemperatur wurde diese über eine Glasfritte (P4) filtriert. Das Kristallisat wurde mit 85 %iger und dann mit 50 %iger Schwefelsäure gewaschen, in Wasser suspendiert, abgesaugt, pH-neutral gewaschen und bei 60 °C im Vakuum getrocknet.
Ausbeute: 8.8 g gelbe Mikrokristalle
Laut Elementaranalyse betrug der Chlorierungsgrad n = 8.2.

| | | | | |
|---|---|---|---|---|
| C₄₄H₂₀₋ₙClₙO₄N₄ | Gef. C 54.3 | H 1.5 | Cl 29.9 | N 5.8 |

Nach dem Massenspektrum (SIMS) lag im Wesentlichen ein Makrocyclengemisch mit fünf bis neun Chloratomen vor (Hauptpeak mit acht Chloratomen bei M⁺ = 944.9).

### Beispiel 23

### α-Amidomethylierung von Cyclo-2,9':2',9'':2'',9''':2''',9-quaternaphtho[1,2-d]-oxazol mit N-Hydroxymethylpyrrolidon

2.50 g (3.73 mmol) Cyclo-2,9':2',9'':2'',9''':2''',9-quaternaphtho[1,2-d]oxazol (CQNO) und 2.58 g (22.4 mmol) N-Hydroxymethylpyrrolidon wurden in 50 ml konz. Schwefelsäure gelöst und 20 Stunden lang bei 70 °C gerührt. Nach dem Abkühlen auf Raumtemperatur wurde die Lösung auf eine Eis-Wasser-Mischung gefällt. Der Niederschlag wurde abgesaugt und mit Wasser pH-neutral gewaschen. Das Nutschgut wurde in Wasser suspendiert und mit Ammoniaklösung auf pH 9 gestellt. Der Feststoff wurde abgesaugt, mit Wasser gewaschen und bei 60 °C im Vakuum getrocknet.
Ausbeute: 2.60 g gelbes Pulver

### Beispiel 24

### α-Amidomethylierung von Cyclo-2,9':2',9'':2'',9''':2''',9-quaternaphtho[1,2-d]-oxazol mit Caprolactam/Formaldehyd

3.34 g (5.0 mmol) Cyclo-2,9':2',9'':2'',9''':2''',9-quaternaphtho[1,2-d]oxazol (CQNO), 0.9 g (30 mmol) Paraformaldehyd und 3.4 g (30 mmol) Caprolactam werden nacheinander unter Rühren in 100 g 85 %ige Polyphosphorsäure bei 40 - 45 °C gelöst. Die Reaktionsmischung wird auf 105 °C erhitzt und 8.5 Stunden bei dieser Temperatur gerührt. Nach dem Abkühlen auf 90 °C wird die Reaktionslösung auf Eis/Wasser gefällt und unter Kühlung (Eiszugabe) mit konz. Ammoniaklösung auf pH 8.5 gestellt. Der Feststoff wurde abgesaugt, mit Wasser salzfrei gewaschen und bei 60 °C im Vakuum getrocknet.
Ausbeute: 4.9 g gelbes Pulver

Das in Essigsäure lösliche Produktgemisch enthielt laut MALDI-Massenspektrum neben unbekannten Verbindungen CQNO mit ein bis fünfN-Methylen-caprolactamresten
([M+H]⁺ = 794.2, 919.3, 1044.4, 1169.4, 1294.6).

### Beispiel 25

### α-Amidomethylierung von Cyclo-2,9':2',9'':2'',9''':2''',9-quaternaphtho[1,2-d]-oxazol mit N-Hydroxymethyl-5-t-butyl-caprolactam

3.34 g (5.0 mmol) Cyclo-2,9':2',9'':2'',9''':2''',9-quaternaphtho[1,2-d]oxazol (CQNO), 5.97 g (30 mmol) 5-t-Butylcaprolactam werden nacheinander unter Rühren in 107 g 85 %ige Polyphosphorsäure bei 40 - 45 °C gelöst. Die Reaktionsmischung wird auf 105 °C erhitzt und 8 Stunden bei dieser Temperatur gerührt. Nach dem Abkühlen auf 90 °C wird die Reaktionslösung auf Eis/ Wasser gefällt und unter Kühlung (Eiszugabe) mit konz. Ammoniaklösung auf pH 8.5 gestellt. Der Feststoff wurde abgesaugt, mit Wasser salzfrei gewaschen und bei 60 °C im Vakuum getrocknet.
Ausbeute: 7.2 g gelbes Pulver
Das Produktgemisch war in Tetrahydrofuran (THF) löslich.
UV/Vis: λₘₐₓ = 400, 324, 270, 220 nm in Schwefelsäure,
λₘₐₓ = 370, 320, 266 nm in THF

### Beispiel 26

### Imidazolylmethylierung von Cyclo-2,9':2',9'':2'',9''':2''',9-quaternaphtho[1,2-d]-oxazol mit Imidazol/Formaldehyd

1.34 g (44.8 mmol) Paraformaldehyd und 3.06 g (45.0 mmol) Imidazol wurden gleichzeitig in 75 ml 97 %ige Schwefelsäure unter Rühren bei Raumtemperatur eingetragen, wobei die Reaktionstemperatur 50 °C nicht überschreiten sollte. Anschließend wurde die Reaktionslösung auf 80 °C erwärmt und 70 min bei dieser Temperatur gerührt. Sobald kein Formaldehyd mehr nachweisbar war, wurden bei 58 - 60 °C 5.00 g (7.47 mmol) Cyclo-2,9':2',9'':2'',9''':2''',9-quaternaphtho[1,2-d]oxazol (CQNO) innerhalb von einer Stunde zugegeben. Die Lösung wurde 165 min lang bei 60 °C gerührt, dann auf 100 °C erhitzt und drei Stunden bei dieser Temperatur gehalten. Nach dem Abkühlen auf Raumtemperatur wurde die Lösung auf eine Eis-Wasser-Mischung gefällt und mit Ammoniaklösung auf pH 9 gestellt, wobei die Temperatur durch Eiszugabe unter 20 °C gehalten wurde. Der Feststoff wurde abgesaugt, mit Wasser pH-neutral und salzfrei gewaschen und bei 60 °C im Vakuum getrocknet.
Ausbeute: 6.5 g gelbes Pulver
UV/Vis: λₘₐₓ = 406, 326, 270, 220 nm in Schwefelsäure,
λₘₐₓ = 368, 320, 270 nm in Essigsäure
Laut MALDI-MS lag ein Produktgemisch aus CQNO mit n = 1 - 6
Methylenimidazolresten vor (höchster Peak bei 909.3 von CQNO mit n = 3).

### Beispiel 27

### Imidazolylmethylierung von Cyclo-2,9':2',9'':2'',9''':2''',9-quaternaphtho[1,2-d]-oxazol mit N-Methylimidazol/Formaldehyd

1.34 g (44.8 mmol) Paraformaldehyd und 3.69 g (45.0 mmol) N-Methylimidazol wurden gleichzeitig in 75 ml 97 %ige Schwefelsäure unter Rühren bei Raumtemperatur eingetragen bzw. zugetropft, wobei die Reaktionstemperatur 50 °C nicht überschreiten sollte. Anschließend wurde die Reaktionslösung auf 80 °C erwärmt und 70 min bei dieser Temperatur gerührt. Sobald kein Formaldehyd mehr nachweisbar war, wurden bei 58 - 60 °C 5.00 g (7.47 mmol) Cyclo-2,9':2',9":2",9''':2''',9-quaternaphtho[1,2-d]oxazol (CQNO) innerhalb von einer Stunde zugegeben. Die Lösung wurde 165 min lang bei 60 °C gerührt, dann auf 100 °C erhitzt und drei Stunden bei dieser Temperatur gehalten. Nach dem Abkühlen auf Raumtemperatur wurde die Lösung auf eine Eis-Wasser-Mischung gefällt und mit Ammoniaklösung auf pH 9 gestellt, wobei die Temperatur durch Eiszugabe unter 20 °C gehalten wurde. Der Feststoff wurde abgesaugt, mit Wasser pH-neutral und salzfrei gewaschen und bei 60 °C im Vakuum getrocknet.
Ausbeute: 7.0 g gelbes Pulver
UV/Vis: λₘₐₓ = 368, 320, 270 nm in Essigsäure
Laut MALDI-MS lag ein Produktgemisch aus CQNO mit n = 1 - 5 Methylen-N-methylimidazolresten vor (höchster Peak bei 951.3 von CQNO mit n = 3).

### Beispiel 28

### 4-Amino-7-brom-3-hydroxy-2-naphthoesäure

Azokupplung: 4.37 g (25.0 mmol) 99 %ige Sulfanilsäure wurden durch Zugabe von 2.09 g 50 %iger Natronlauge in 70 ml Wasser unter Rühren gelöst. Nach der Zugabe einer Lösung von 1.73 g (25.0 mmol) Natriumnitrit in Wasser wurde die Reaktionslösung auf Nitrit geprüft (negativ), mit weiteren 0.33 g (4.8 mmol) Natriumnitrit versetzt und dann langsam zu einer Mischung aus 100 g Eis/Wasser und 9.70 g (98.4 mmol) konz. HCl dosiert. Nach 2stündigem Rühren bei 0 - 5 °C wurde der Nitritüberschuss durch Zugabe von Amidosulfonsäure vernichtet. Die so erhaltene Diazoniumsalzlösung wurde zu einer Lösung (pH 9 - 9.5) von 6.68 g (25.0 mmol) 7-Brom-3-hydroxy-2-naphthoesäure und 1.48 g 50 % NaOH in 200 ml Wasser/Acetonitril (1 : 1) dosiert, wobei durch Zugabe von 7.45 g 50 % NaOH der pH-Wert 8 - 9 gehalten wurde. Die Azofarbstofflösung wurde eine Stunde lang nachgerührt.

Reduktion: Die Lösung des Azofarbstoffs wurde mit 1.34 g (16.8 mmol) 50 % NaOH unter Rühren versetzt (pH 8.9) und unter Stickstoff auf 80 °C erhitzt. 8.71 g (50.0 mmol) Natriumdithionit wurden portionsweise zugegeben, wobei der pH-Wert der Lösung durch Zugabe von 2.77 g 50 % NaOH über 8 gehalten wurde. Nach Entfärben der Reaktionslösung ließ man diesen auf Raumtemperatur abkühlen und stellte mit konz. HCl auf pH 5.1 ein, wobei ein Niederschlag ausfiel. Der Feststoff wurde unter Stickstoff abgesaugt, mit Wasser gewaschen und im Exsikkator über NaOH getrocknet.
Ausbeute: 5.13 g fast farbloses Pulver; Schmp. 264 - 267 °C (Zers.)
¹H-NMR (D₆-DMSO): 7.50 (d; 1H, Aromaten-H), 7.82 (s; 1H, Aromaten-H), 8.00
(d; 1H, Aromaten-H), 8.10 (s; 1H, Aromaten-H), 9.1 (breit; 2H)

### Beispiel 29

### 3,9,15,21-Tetrabrom-cyclo-2,9':2',9'':2'',9''':2''',9-quaternaphtho[1,2-d]oxazol

In 100.9 g 135 °C heiße 85 %ige Polyphosphorsäure wurden unter Rühren und Stickstoff 4.23 g (15.0 mmol) 4-Amino-7-brom-3-hydroxy-2-naphthoesäure portionsweise eingetragen. Die Reaktionslösung wurde auf 150 °C erhitzt und 24 Stunden bei dieser Temperatur gerührt. Nach Abkühlen der Lösung auf 120 °C wurde diese unter Rühren zu 250 g Eis gegeben, wobei ein Niederschlag ausfiel. Die Suspension wurde mit 161 g konz. Ammoniaklösung neutralisiert, wobei durch Zugabe von 127 g Eis gekühlt wurde. Der Niederschlag wurde abgesaugt und solange mit Wasser gewaschen, bis das Waschwasser pH-neutral reagierte. Das Rohprodukt wurde bei 75 °C im Vakuum getrocknet (3.62 g). Zur Reinigung wurde das braune Rohprodukt in 127 g 96 - 98 %ige Schwefelsäure gelöst. Die Lösung wurde über eine Glasfritte filtriert und langsam mit 16.5 g 50 %iger Schwefelsäure versetzt, wobei sich ein Kristallisat bildete. Der Feststoff wurde über eine Glasfritte abgesaugt, mit 90 %iger Schwefelsäure und mit Wasser gewaschen und bei 75 °C im Vakuum getrocknet (1.25 g). In einem zweiten Reinigungsschritt wurde der Feststoff in 29.3 g 100 %iger Schwefelsäure gelöst und langsam mit 0.59 g 95 - 97 %iger Schwefelsäure und dann mit 1.73 g 50 %iger Schwefelsäure versetzt. Das Kristallisat wurde über eine Glasfritte abgesaugt, mit Wasser gewaschen und im Vakuum bei 80 °C getrocknet.
Ausbeute: 0.50 g gelbe Mikrokristalle
UV/Vis: λₘₐₓ = 410, 332, 262,228 nm in konz. Schwefelsäure

### Beispiel 30

### Anwendung als Pigmentdispergator

Perylen-3,4-dicarbonsäureimid-9-carbonsäure wird gemäß CAS 75:140549j (1971) hergestellt. Als geeignete Kupferphthalocyaninsulfonsäure wird Solsperse 12000 (Fa. Avecia) verwendet. Als Hyperdispersant verwendet man Solsperse 24000.
a) 7.50 g eines transparenten handelsüblichen Perylenpigments wie zum Beispiel P.R.179 (L3885 BASF) werden jeweils mit 225 mg der Synergisten Perylen-3,4-dicarbonsäureimid-9-carbonsäure (Probe 1), mit 225 mg Solsperse 12000 (Probe 2) und mit 225 mg sulfoniertem Cyclo-2,9':2',9":2'',9''':2''',9-quaternaphtho[1,2-d]oxazol aus Beispiel 21 (Probe 3) intensiv miteinander trocken gemischt. Als Vergleich werden 7.725 g eines Pigments ohne Synergisten eingesetzt (Probe 4). Die Dispergierung erfolgt in einer 100 ml Glasflasche unter Verwendung von 27 ml Glaskugeln mit einem Kugeldurchmesser von 3 mm. Nach Zugabe von 1.2 g Hyperdispersant Solsperse 24000 und 23.3 g eines High Solid Lacks (Acrylharz mit Xylol und Butylacetat 1:1 als Lösungsmittel, Festkörpergehalt 45%) zu sämtlichen Proben wird 2 Stunden mit einem Skandex Dispergieraggregat geschüttelt. Man lässt die Pasten 24 Stunden stehen. Die Pasten 1a - 3a weisen ein dünnflüssige Konsistenz auf, während die Vergleichspaste 4a eine dickflüssige Konsistenz aufweist. Die Synergisten haben eine ähnliche Wirksamkeit. Nach Mischen von je 1.0 g Lackpaste mit 2.0 g einer Weißpaste (aus 20 g TiO₂ dispergiert in 60 g unpigmentierten High-Solid-Lack s. oben) und Aufrakeln dieser Paste auf ein Blech und anschließendes Einbrennen (30 Minuten, 130°C) werden Lackfilme erhalten. Die Lackfilme aus 1a, 3a und 4a weisen eine ähnliche Farbe auf, während der Film aus Paste 2a (enthält blaues Phthalocyaninderivat) deutlich trüber und blauer ist.
b) Man wiederholt das oben genannte Experiment unter Verwendung von 10.0 g eines handelsüblichen Pigmentblaus wie beispielsweise P.B. 15:1 (L6930 BASF) an Stelle von 7.5 g P.R.179. Die Pasten 1b - 3b weisen eine ähnlich gute, dünnflüssige Fließfähigkeit auf. Nach Erstellen der Lackfilme ist zu erkennen, dass der Film aus Paste 1b (enthält Perylenderivat) röter und trüber erscheint, während die übrigen Filme aus den Pasten 2b, 3b, 4b visuell betrachtet einen annähernd identischen Farbeindruck hervorrufen.

### Beispiel 31

Organische Schichten werden im Hochvakuum (10⁻⁶ mbar) auf ein sauberes mit Indium-Zinn-Oxid (ITO) beschichtetes Glas aufgedampft. Auf die ITO-Schicht (Anode) werden nacheinander eine Lochleiterschicht aus N,N'-Diphenyl-N,N'-bis(3-methylphenyl)-(1,1'-biphenyl)-4,4'-diamin (TPD), eine Emitterschicht aus Cyclo-2,4':2',7'':2'',4''':2''',7-quaterbenzimidazol (CQBI), eine Elektronenleiterschicht aus Aluminium-tri-(8-chinolinolat) (Alq₃) und eine Kathodenschicht aus Aluminium aufgedampft.

### Beispiel 32

Organische Schichten werden im Hochvakuum (10⁻⁶ mbar) auf ein sauberes mit Indium-Zinn-Oxid (ITO) beschichtetes Glas aufgedampft. Auf die ITO-Schicht (Anode) werden nacheinander eine Lochleiterschicht aus N,N'-Di-(1-naphthyl)-N,N'-diphenyl-4,4'-benzidin (α-NPB), eine Emitterschicht, bestehend aus dem Matrixmaterial 4,4'-(Biphenyl)-N,N'-dicarbazol (CBP) und dem Emitter Platin-cyclo-2,4':2',7'':2'',4''':2''',7-quaterbenzimidazol (Pt-CQBI) durch Coverdampfung, eine Lochblockerschicht aus 2,9-Dimethyl-4,7-diphenyl-phenanthrolin (BCP), eine Elektronenleiterschicht aus Aluminium-tri-(8-chinolinolat) (Alq₃), eine Elektroneninjektionsschicht aus Lithiumfluorid und eine Kathodenschicht aus Aluminium aufgedampft.

### Beispiel 33

Organische Schichten werden im Hochvakuum (10⁻⁶ mbar) auf ein sauberes mit Indium-Zinn-Oxid (ITO) beschichtetes Glas aufgedampft. Auf die ITO-Schicht (Anode) werden nacheinander eine Lochinjektionsschicht aus Kupferphthalocyanin (CuPc), eine Lochleiterschicht aus N,N'-Di-(1-naphthyl)-N,N'-diphenyl-4,4'-benzidin (α-NPB), eine Emitterschicht, bestehend aus dem Matrixmaterial 4,4'-(Biphenyl)-N,N'-dicarbazol (CBP) und dem Emitter Platin-cyclo-2,4':2',7'':2'',4''':2''',7-quaterbenzimidazol (Pt-CQBI) durch Coverdampfung, eine Lochblockerschicht aus 2,9-Dimethyl-4,7-diphenylphenanthrolin (BCP), eine Elektronenleiterschicht aus Aluminium-tri-(8-chinolinolat) (Alq₃), eine Elektroneninjektionsschicht aus Lithiumfluorid und eine Kathodenschicht aus Aluminium aufgedampft.

### Beispiel 34

Organische Schichten werden im Hochvakuum (10⁻⁶ mbar) auf ein sauberes mit Indium-Zinn-Oxid (ITO) beschichtetes Glas aufgedampft. Auf die ITO-Schicht (Anode) werden nacheinander eine Lochinjektionsschicht aus Cyclo-2,9':2',9'':2'',9''':2''',9-quatemaphtho[1,2-d]oxazol, eine Lochleiterschicht aus N,N'-Di-(1-naphthyl)-N,N'-diphenyl-4,4'-benzidin (α-NPB), eine Emitterschicht, bestehend aus dem Matrixmaterial 4,4'-(Biphenyl)-N,N'-dicarbazol (CBP) und dem Emitter Platin-cyclo-2,4':2',7'':2'',4''':2''',7-quaterbenzimidazol (Pt-CQBI) durch Coverdampfung, eine Lochblockerschicht aus 2,9-Dimethyl-4,7-diphenylphenanthrolin (BCP), eine Elektronenleiterschicht aus Aluminium-tri-(8-chinolinolat) (Alq₃), eine Elektroneninjektionsschicht aus Lithiumfluorid und eine Kathodenschicht aus Aluminium aufgedampft.

### Beispiel 35

Organische Schichten werden im Hochvakuum (10⁻⁶ mbar) auf ein sauberes mit Indium-Zinn-Oxid (ITO) beschichtetes Glas aufgedampft. Auf die ITO-Schicht (Anode) werden nacheinander eine Lochinjektionsschicht aus Cyclo-2,9':2',9'':2'',9''':2''',9-quatemaphtho[1,2-d]oxazol, eine Lochleiterschicht aus N,N'-Di-(1-naphthyl)-N,N'-diphenyl-4,4'-benzidin (α-NPB), eine Emitterschicht, bestehend aus dem Matrixmaterial 4,4'-(Biphenyl)-N,N'-dicarbazol (CBP) und dem Emitter Iridium-tris(2-phenylpyridin) (Ir(ppy)₃) durch Coverdampfung, eine Lochblockerschicht aus 2,9-Dimethyl-4,7-diphenylphenanthrolin (BCP), eine Elektronenleiterschicht aus Aluminium-tri-(8-chinolinolat) (Alq₃), eine Elektroneninjektionsschicht aus Lithiumfluorid und eine Kathodenschicht aus Aluminium aufgedampft.

### Beispiel 36

Anwendungsbeispiel für Verbindungen aus Beispielen 13 beziehungsweise 15 gegenüber dem pigmentären UV-Absorber ZnO (Z-Cote; BASF)

Als pigmentäre anorganische UV-Absorber für Kosmetik, Lack und Kunststoff gibt es nanopartikuläres ZnO (DE-A 19907704, EP-A 0449888). Diese haben jedoch den Nachteil, dass sie bei ungenügender Feinheit weißes Licht streuen, so dass ein milchiges Aussehen hervorgerufen wird. Ungenügende Feinheit tritt bei zu großer Partikelgröße auf, wobei entweder die Primärpartikel zu groß sind oder der Dispergierzustand nicht ausreichend ist. Die beanspruchten Verbindungen weisen ein ähnliches Absorptionsspektrum wie ZnO auf, streuen jedoch als organische Pigmente in den beschriebenen Formulierungen weniger.

### In LDPE

In einem Mini Extruder (MiniLab Micro Rheology Compounder , Fa. Thermo Haake) wurden 5 g LDPE 1840 (Fa. Basell) und 0.05 g der Proben aus den Beispielen 1d, 2 und kommerzielles Zinkoxid (Z-Cote, Fa. BASF) bei 170°C plastifiziert und 5 Minuten bei dieser Temperatur dispergiert. Der Austrag wurde mittels einer 180°C heißen Spritzpistole in ein Werkzeug der Temperatur 50°C (2.5 cm Durchmesser, 1.5 mm Dicke) eingetragen. Dieser Spritzling wurde auf eine Dicke von 0.25 mm verpresst. Schließlich wurde der Spritzling bei 180°C mit einer Dampfpresse (Fa. Wickert) zwischen zwei Pressplatten auf 0.25 mm Stärke gepresst und visuell und UV-spektroskopiesch charakterisiert.
Die Presslinge, welche die Verbindungen aus Beispiel 1 und 2 enthalten, wiesen visuell eine höhere Transparenz auf als die ZnO enthaltende Probe bei ansonsten ähnlicher Absorptionsstärke.

### Im Automobil-Klarlack

60 mg der Verbindungen aus Beispiel 1 und 2, sowie Zinkoxid (Z-Cote, BASF) wurden in einer 10 ml Glasflasche mit 6 g SAZ-Kugeln (1-1.6 mm Durchmesser) und 5.94 g eines Automobilklarlackes ohne UV-Absorber (Duraclear II, BASF-Coatings, 50% Festkörpergehalt, Lsm. Butylacetat) 60 Minuten auf einem Skandex Dispergieraggregat (Fa. Lau) bei Stufe 2 dispergiert. Mit einem Spiralhandrakel wurde ein 150µm Film aufgetragen und 30 Minuten bei 130°C eingebrannt. Die Filme wurden visuell beurteilt. Die Beispiele 1 und 2 enthaltenden Verbindungen wiesen bei ähnlicher Absorption eine geringere Streuung von weißem Licht auf, welches sich in einem weniger milchig wirkendem Aussehen bemerkbar macht.

### In einer kosmetischen Formulierung

8.00 g Dibutyladipate (Cetiol b, Fa. Cognis), 8.00 g C₁₂₋₁₅ Alkylbenzoate 12.00 g Cocogylceride (Myritol 331, Fa. CA Erbslöh), 1.00g Natrium Cetearyl Sulfate (Lanette E, Fa. Cognis) und 4.00g Lauryl Glucoside, (Eumulgin VL75, Fa. Cognis), 2.00 g Cetearyl Alcohol (Lanette 0) und 1.00 g Vitamin E Acetat (BASF) und 3.00 g Ethylhexyl-triazon (Uvinul T150 BASF) werden bei 80°C homogenisiert.
Danach wurden bei dieser Temperatur die erfindungsgemäßen Verbindungen aus Beispiel 1 und 2, sowie Z-Cote zugesetzt und mit einem Ultrathurax-Rührstab 3 Minuten dispergiert. Zu dieser Dispersion wurde eine 80°C warme Dispersion gegeben, welche durch Homogenisieren von 3.00 g Glycerin, 0.05 g EDTANa₂ (EDETA BD, Fa. BASF), 0.20 g Allantoin, 0.30 g Xanthan Gummi (Keltrol, Fa. Kleco), 1.50 g Magnesium Aluminium Silikat (Veegum Ultra, Fa. Vanderbilt) und 50.45 g destilliertes Wasser bei 80°C erhalten wurde. Die vereinigten Dispersionen wurden auf 40°C abgekühlt und 0.50 g Zitronensäure, eventuell Duftstoffe und 1.00 g eines Gemisches aus Phenoxyethanol und Alkylparabenen (Phenonip, Fa. Nipa) zugesetzt.
Die erhaltene Formulierung kann als Sonnencreme verwendet werden.
Die Formulierungen, welche die beanspruchten Verbindungen enthielten, wiesen bei gleicher Schichtdicke einen ähnliches Absorptionsspektrum auf, sind jedoch weniger weiß streuend als die ZnO (Z-Cote, BASF) enthaltende Formulierung, was insbesondere bei dunkel pigmentierter Haut ästhetische Vorteile bringt.

## Patentansprüche

1. Verwendung von cyclischen Verbindungen der allgemeinen Formel (I) 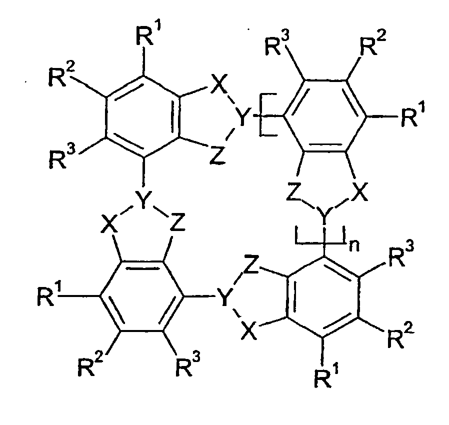 mit der Bedeutung
n Zahl im Bereich von 1 bis 7,
X-Y-Z jeweils unabhängig O-C=N, N=C-O, NR⁵-C=N, N=C-NR⁵, N⁺R⁵₂-C=N, N=C-N⁺R⁵₂, O-C=N⁺R⁵, N⁺R⁵=C-O, S-C=N⁺R⁵, N⁺R⁵=C-S, S-C=N, N=C-S,
R¹, R², R³ jeweils unabhängig H oder Substituent der Gruppe C₁₋₁₂-Alkyl, C₁₋₁₂-Alkanoyl, C₃₋₇-Cycloalkyl, C₆₋₁₂-Aryl, C₇₋₁₃-Aralkyl, C₇₋₁₃-Alkaryl, C₁₋₁₂-Alkoxy, C₆₋₁₂-Aryloxy, C₁₋₁₂-Hydroxyalkyl, Heterocyclus, C₆₋₁₂-Aroyl, die jeweils substituiert sein können, Hydroxy, Thiol, Halogen, Cyan, lsocyan, Nitro, Ammonium, Amino, Phosphin, Phosphinoxid, Sulfonsäure oder Derivat davon, Carbonsäure oder Derivat davon, Derivate des Siliziums, C₂₋₁₂-Alkinyl oder C₂₋₁₂-Alkenyl, wobei die Doppel- oder Dreifachbindungen direkt an das Cycloquatergerüst gebunden sein können oder in der Kette stehen können, Carbamate der Formel -NH-CO-OR⁷, substituierte Harnstoffe der Formel -NR⁷-CO-NR⁷₂, Alkylcarbonat-Substituenten der Formel -O-CO-OR⁷, Sulfinsäure der Formel -SO-OR⁷ sowie Derivaten davon, Sulfoxide der Formel -SO-R⁷ sowie Derivate davon, Phosphonsäure,-salz, -ester- oder -amid davon,
wobei auch R¹ und R² und/oder R² und R³ jeweils unabhängig voneinander gegebenenfalls substituierte anellierte Ringsysteme aus 1 bis 3 Ringen bilden können, die Heteroatomgruppen enthalten können, oder gegebenenfalls substituierte Alkylengruppen bilden können, die durch Heteroatomgruppen unterbrochen sein können, wobei die anellierten Verbindungen auch wie vorstehend für die Reste R¹, R², R³ angegeben substituiert sein können,
wobei in Sauerstoffatome tragenden Resten diese auch durch Schwefelatome ersetzt sein können,
wobei im Mittel 0,05 bis 100% der im Molekül vorliegenden Reste R¹, R², R³ von Wasserstoff verschieden sein können,
oder entsprechenden heterocyclischen Verbindungen, in denen mindestens eine Gruppe -CR¹=, -CR²=, -CR³-CR³= durch -N= ersetzt ist
R⁵ jeweils unabhängig H, gegebenenfalls substituiertes C₁₋₁₂-Alkyl, C₆₋₁₂-Aryl, C₇₋₁₃-Alkylaryl, gegebenenfalls substituiertes C₁₋₁₂-Alkanoyl, gegebenenfalls substituiertes C₇₋₁₃-Aryloyl, Oligoethylenglycol mit 1 bis 6 Sauerstoffatomen, Oligoethylengtycolether mit 1 bis 6 Sauerstoffatomen, Imidazoylmethyl oder ein entsprechender Rest, in dem ein N-Atom durch einen C₁₋₁₂-Alkylrest substituiert und gegebenenfalls positiv geladen ist und eine C-H Gruppe im Ring durch C-(C₁₋₁₂-Alkyl) ersetzt sein kann, (1-C₄₋₆-Lactam)methyl, das am Ring C₁₋₁₂-Alkyl-substituiert sein kann,
R⁷ jeweils unabhängig H, C₁₋₁₂-Alkyl, C₆₋₁₂-Aryl
sowie Tautomerenstrukturen davon
oder Metallkomplexen der cyclischen Verbindungen oder Komplexen der cyclischen Verbindungen mit Mineralsäuren,
wobei bei kationischen Cyclen als Gegenionen X⁻ Chlorid, Sulfat, Hydrogensulfat, Phosphat, Hydrogenphosphat, Nitrat, BF₄⁻, Methansulfonat vorliegen,
als Lichtabsorber, Materialien für Löcherinjektionsschichten in OLEDs, als lichtemittierende Verbindungen in OLED, als Synergisten zur Dispergierung von Pigmenten oder zur optischen Datenspeicherung.

2. Verwendung nach Anspruch 1, **dadurch gekennzeichnet, dass** cyclische Verbindungen der allgemeinen Formel (I) 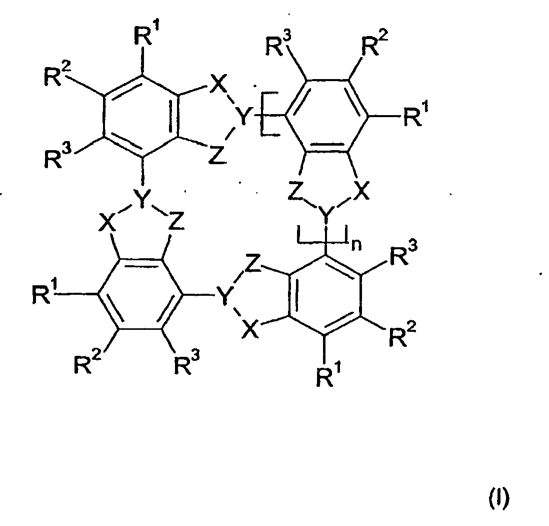 mit der Bedeutung
n ganze Zahl im Bereich von 1 bis 7,
X-Y-Z jeweils unabhängig O-C=N, N=C-O, NH-C=N, N=C-NH, S-C=N. N=C-S,
R¹, R², R³ jeweils unabhängig H oder Substituent der Gruppe C₁₋₁₂-Alkyl, C₁₋₁₂-Alkanoyl, C₃₋₇-Cycloalkyl, C₆₋₁₂-Aryl, C₇₋₁₃-Aralkyl, C₇₋₁₃-Alkaryl, C₁₋₁₂-Alkoxy, C₆₋₁₂-Aryloxy, C₁₋₁₂-Hydroxyalkyl, Heterocyclus, C₆₋₁₂-Aroyl, die jeweils substituiert sein können, Hydroxy, Thiol, Halogen, Cyan, Isocyan, Nitro, Ammonium, Amino, Phosphin, Phosphinoxid, Sulfonsäure oder Derivat davon, Carbonsäure oder Derivat davon, Derivate des Siliziums,
wobei auch R¹ und R² und/oder R² und R³ jeweils unabhängig voneinander gegebenenfalls substituierte anellierte Ringsysteme aus 1 bis 3 Ringen bilden können, die Heteroatomgruppen enthalten können, oder gegebenenfalls substituierte Alkylengruppen bilden können, die durch Heteroatomgruppen unterbrochen sein können,
wobei im Mittel 0,01 bis 12 der im Molekül vorliegenden Reste R¹, R², R³ von Wasserstoff verschieden sein können,
oder entsprechenden heterocyclischen Verbindungen, in denen mindestens eine Gruppe -CR¹=, -CR²=, -CR³ durch -N= ersetzt ist,
oder Metallkomplexen der cyclischen Verbindungen,
als Lichtabsorber, Materialien für Löcherinjektionsschichten in organischen licht-emittierenden Dioden (OLED)
oder als Synergisten zur Dispergierung von Pigmenten eingesetzt werden.

3. Verwendung nach Anspruch 1 oder 2, **dadurch gekennzeichnet, dass** der Lichtabsorber ein UV-Absorber und/oder Vis-Absorber ist.

4. Verwendung nach einem der Ansprüche 1 bis 3, **dadurch gekennzeichnet, dass** die cyclischen Verbindungen der allgemeinen Formel (I) in einem Anwendungsmedium in löslicher, teillöslicher oder nicht-löslicher Form eingesetzt werden, wobei in der nicht-löslichen Form auch Mischkristalle mit anderen Farbmitteln vorliegen können.

5. Verwendung nach einem der Ansprüche 1 bis 4, **dadurch gekennzeichnet, dass** R¹, R², R³ für alle Positionen dieselben Bedeutungen haben.

6. Cyclische Verbindungen oder Metallkomplexe der cyclischen Verbindungen oder Komplexe der cyclischen Verbindungen mit Mineralsäuren, wie sie in Anspruch 1 oder 2 definiert sind, ausgenommen cyclische Verbindungen mit der Bedeutung
X-Y-Z N=C-O, NH-C=N, N=C-NH,
R¹, R², R³ H, C₁₋₆-Alkyl.

7. Verfahren zur Herstellung von cyclischen Verbindungen der allgemeinen Formel (I) gemäß Anspruch 6 durch Cyclisierung von Verbindungen der allgemeinen Formel (II) 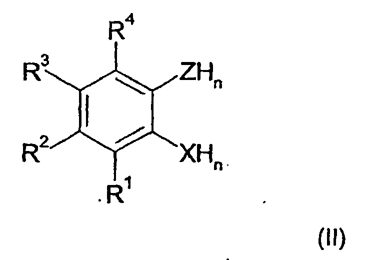 mit der Bedeutung
R¹, R², R³, X, Z wie angegeben
R⁴ -COOH oder Derivat davon,
n jeweils 1 oder 2, zum Erhalt der Stöchiometrie,
wobei OH-Gruppen auch als Alkalimetallsalz- öder Ammoniumsalz-Gruppen vorliegen können und/oder NH₂-Gruppen in protonierter Form oder Derivat-Form als -NO, -NO₂, -N=N-Aryl, =NOH, =NH vorliegen können, wobei die Cyclisierung in Gegenwart von Metallsalzen, Metallpulvern oder von Lewis-Säuren als Templaten und in Gegenwart von Kondensationsmitteln oder unter wasserentziehenden Bedingungen durchgeführt werden kann.

8. Verfahren zur Herstellung von Komplexen cyclischer Verbindungen durch Herstellung der cyclischen Verbindungen nach einem Verfahren gemäß Anspruch 8 in Gegenwart von Metallsalzen, Metallpulvern, als Templaten oder durch Umsetzung von cyclischen Verbindungen gemäß Anspruch 7 mit Metallsalzen oder Metallpulvern.

9. Verwendung nach einem der Ansprüche 1 bis 5 als Lichtabsorber zum Einfärben von hochmolekularen organischen Materialien.

10. Thermoplastische Formmassen, Lacke und Beschichtungszusammensetzungen, enthaltend Lichtabsorber, wie sie in einem der Ansprüche 1 bis 5 definiert sind.

## Claims

1. The use of cyclic compounds of the formula (I) 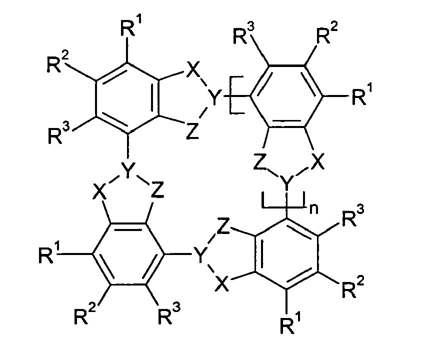 where
n is a number in the range from 1 to 7,
X-Y-Z, in each case independently of one another, is O-C=N, N=C-O, NR⁵-C=N, N=C-NR⁵, N⁺R⁵₂-C=N, N=C-N⁺R⁵₂, O-C=N⁺R⁵, N⁺R⁵=C-O, S-C=N⁺R⁵, N⁺R⁵=C-S, S-C=N, N=C-S,
R¹, R² and R³, in each case independently of one another, are H or a substituent from the group consisting of C₁₋₁₂-alkyl, C₁₋₁₂-alkanoyl, C₃₋₇-cycloalkyl, C₆₋₁₂-aryl, C₇₋₁₃-aralkyl, C₇₋₁₃-alkaryl, C₁₋₁₂-alkoxy, C₆₋₁₂-aryloxy, C₁₋₁₂-hydroxyalkyl, a heterocycle, C₆₋₁₂-aroyl, each of which may be substituted, hydroxyl, thiol, halogen, cyano, isocyano, nitro, ammonium, amino, phosphine, phosphine oxide, a sulfonic acid or a derivative thereof, carboxylic acid or a derivative thereof, a derivative of silicon, C₂₋₁₂-alkynyl or C₂₋₁₂-alkenyl, it being possible for the double or triple bonds to be linked directly to the cycloquater skeleton or to be in the chain, a carbamate of the formula -NH-CO-OR⁷, a substituted urea of the formula -NR⁷-CO-NR⁷₂, an alkyl carbonate substituent of the formula -O-CO-OR⁷, a sulfinic acid of the formula -SO-OR⁷ or a derivative thereof, a sulfoxide of the formula -SO-R⁷ or a derivative thereof, phosphonic acid or a salt, ester or amide thereof,
it also being possible for R¹ and R² and/or R² and R³, in each case independently of one another, to form unsubstituted or substituted fused ring systems comprising from 1 to 3 rings, which may comprise hetero atom groups, or to form unsubstituted or substituted alkylene groups which may be interrupted by hetero atom groups, it also being possible for the fused compounds to be substituted as stated above for the radicals R¹, R² and R³,
it being possible for oxygen atoms in radicals carrying oxygen atoms also to be replaced by sulfur atoms,
it being possible for on average from 0.05 to 100% of the radicals R¹, R² and R³ present in the molecule to differ from hydrogen,
or corresponding heterocyclic compounds in which at least one group -CR¹=, -CR²=, -CR³-CR³= is replaced by -N=,
R⁵, in each case independently of one another, are H, unsubstituted or substituted C₁₋₁₂-alkyl, C₆₋₁₂-aryl, C₇₋₁₃-alkylaryl, unsubstituted or substituted C₁₋₁₂-alkanoyl, unsubstituted or substituted C₇₋₁₃-aroyl, oligoethylene glycol having 1 to 6 oxygen atoms, oligoethylene glycol ether having 1 to 6 oxygen atoms, imidazoylmethyl or a corresponding radical in which a nitrogen atom is substituted by a C₁₋₁₂-alkyl radical and may, if appropriate, carry a positive charge and a C-H group in the ring may be replaced by C-(C₁₋₁₂-alkyl), or (1-C₄₋₆-lactam)methyl, which may be C₁₋₁₂-alkyl-substituted on the ring,
R⁷, in each case independently of one another, are H, C₁₋₁₂-alkyl or C₆₋₁₂-aryl,
and tautomeric structures thereof
or metal complexes of the cyclic compounds or complexes of the cyclic compounds with mineral acids,
chloride, sulfate, bisulfate, phosphate, hydrogen phosphate, nitrate, BF₄⁻ or methanesulfonate being present as opposite ions X- in the case of cationic cyclic structures,
as light absorbers, materials for hole injection layers in OLEDs, light-emitting compounds in OLED and synergistic agents for the dispersing of pigments or for optical data storage.

2. The use according to claim 1, wherein a cyclic compound of the formula (I) 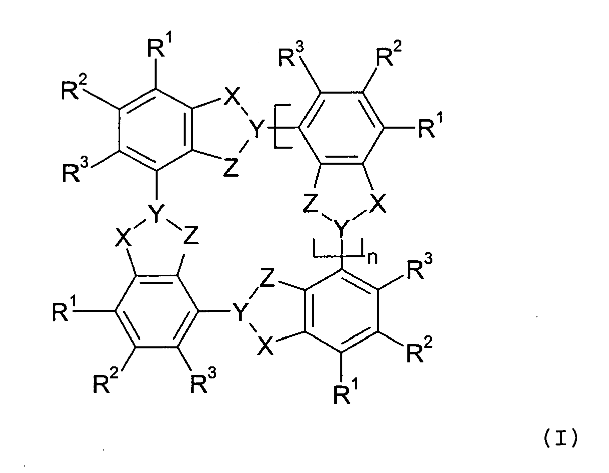 where
n is an integer in the range from 1 to 7,
X-Y-Z, in each case independently of one another, is O-C=N, N=C-O, NH-C=N, N=C-NH, S-C=N or N=C-S,
R¹, R² and R³, in each case independently of one another, are H or a substituent from the group consisting of C₁₋₁₂-alkyl, C₁₋₁₂-alkanoyl, C₃₋₇-cycloalkyl, C₆₋₁₂-aryl, C₇₋₁₃-aralkyl, C₇₋₁₃-alkaryl, C₁₋₁₂-alkoxy, C₆₋₁₂-aryloxy, C₁₋₁₂-hydroxyalkyl, a heterocycle, C₆₋₁₂-aroyl, each of which may be substituted, hydroxyl, thiol, halogen, cyano, isocyano, nitro, ammonium, amino, phosphine, phosphine oxide, a sulfonic acid or a derivative thereof, a carboxylic acid or a derivative thereof or a derivative of silicon,
it also being possible for R¹ and R² and/or R² and R³, in each case independently of one another, to form unsubstituted or substituted fused ring systems comprising from 1 to 3 rings, which may comprise hetero atom groups, or to form unsubstituted or substituted alkylene groups which may be interrupted by hetero atom groups,
it being possible on average for from 0.01 to 12 of the radicals R¹, R² and R³ present in the molecule to differ from hydrogen,
or corresponding heterocyclic compounds in which at least one group -CR¹=, -CR²= or -CR³ is replaced by -N=,
or metal complexes of the cyclic compounds,
as light absorbers, materials for hole injection layers in organic light-emitting diodes (OLED)
or as synergistic agents for the dispersing of pigments,
is used.

3. The use according to claim 1 or 2, wherein the light absorber is a UV absorber and/or Vis absorber.

4. The use according to any of claims 1 to 3, wherein the cyclic compound of the formula (I) is used in soluble, partly soluble or insoluble form in an application medium, it also being possible for solid solutions with other colorants to be present in the insoluble form.

5. The use according to any of claims 1 to 4, wherein R¹, R² R³ have the same meanings for all positions.

6. A cyclic compound or a metal complex of the cyclic compounds or a complex of the cyclic compounds with a mineral acid, as defined in claim 1 or 2, with the exception of cyclic compounds where
X-Y-Z is N=C-O, NH-C=N or N=C-NH,
R¹, R² and R³ are H or C₁₋₆-alkyl.

7. A process for the preparation of a cyclic compound of the formula (I) according to claim 6 by cyclization of a compound of the formula (II) 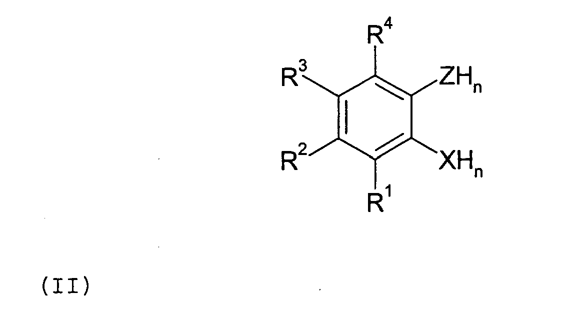 where
R¹, R², R³, X and Z are as stated,
R⁴ is -COOH or a derivative thereof and
n in each case is 1 or 2, to obtain the stoichiometry,
it also being possible for OH groups to be present as alkali metal salt or ammonium salt groups and/or for NH₂ groups to be present in protonated form or derivative form as -NO, -NO₂, -N=N-aryl, =NOH, =NH, and it being possible for the cyclization to be carried out in the presence of metal salts, metal powders or Lewis acids as templates and in the presence of condensing agents or under dehydrating conditions.

8. A process for the preparation of a complex of a cyclic compound by the preparation of the cyclic compound by a process according to claim 7 in the presence of metal salts or metal powders as templates or by reaction of a cyclic compound according to claim 7 with a metal salt or metal powder.

9. The use according to any of claims 1 to 5 as a light absorber for coloring high molecular weight organic materials.

10. A thermoplastic molding material, finish or coating composition comprising a light absorber as defined in any of claims 1 to 5.

## Revendications

1. Utilisation de composés cycliques de formule générale (I) : 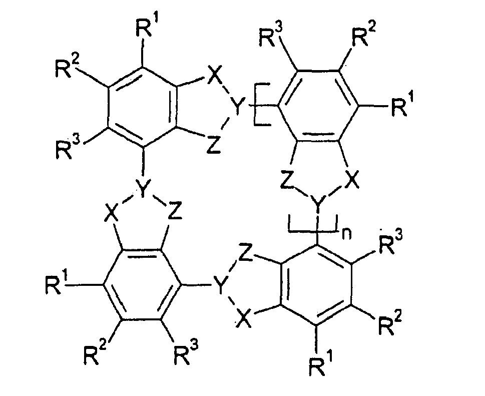 avec les significations suivantes :
n est un nombre dans la plage de 1 à 7,
X-Y-Z représente chaque fois indépendamment O-C=N, N=C-O, NR⁵-C=N, N=C-NR⁵, N⁺R⁵₂-C=N, N=C-N⁺R⁵₂, O-C=N⁺R⁵, N⁺R⁵=C-O, S-C=N⁺R⁵, N⁺R⁵=C-S, S-C=N, N=C-S,
R¹, R² et R³ représentent chaque fois indépendamment H ou un substituant du groupe comprenant les groupements alkyle en C₁₋₁₂, alcanoyle en C₁₋₁₂, cycloalkyle en C₃₋₇, aryle en C₆₋₁₂, aralkyle en C₇₋₁₃, alkaryle en C₇₋₁₃, alcoxy en C₁₋₁₂, aryloxy en C₆₋₁₂, hydroxyalkyle en C₁₋₁₂, un hétérocycle, aroyle en C₆₋₁₂, chacun pouvant être substitué, hydroxy, thiol, halogène, cyano, isocyano, nitro, ammonium, amino, phosphine, oxyde de phosphine, un acide sulfonique ou un de ses dérivés, un acide carboxylique ou un de ses dérivés, des dérivés du silicium, alcynyle en C₂₋₁₂ ou alcényle en C₂₋₁₂, les doubles ou triples liaisons pouvant être reliées directement à l'ossature cyclique à quatre structures ou se situer dans la chaîne, des carbamates de formule -NH-CO-OR⁷, des urées substituées de formule -NR⁷-CO-NR⁷₂, des substituants alkylcarbonate de formule -O-CO-OR⁷, un acide sulfinique de formule -SO-OR⁷ ainsi que ses dérivés, des sulfoxydes de formule -SO-R⁷ et leurs dérivés, l'acide phosphonique, un sel, un ester ou un amide de celui-ci,
dans laquelle également R¹ et R² et/ou R² et R³ peuvent former, chaque fois indépendamment, des systèmes cycliques condensés, éventuellement substitués, composés de 1 à 3 cycles pouvant contenir des groupes d'hétéroatomes, ou peuvent former des groupements alkylène éventuellement substitués, qui peuvent être interrompus par des groupes d'hétéroatomes, les composés condensés pouvant également être substitués comme indiqué précédemment pour les radicaux R¹, R², R³,
où l'on peut, dans les radicaux portant des atomes d'oxygène, également remplacer ceux-ci par des atomes de soufre,
dans laquelle, en moyenne, 0,05 à 100% des radicaux R¹, R² et R³ présents dans la molécule peuvent être différents de l'hydrogène,
ou des composés hétérocycliques correspondants, dans lesquels au moins un groupement -CR¹=, -CR²=, -CR³-CR³= est remplacé par -N=,
R⁵ représente chaque fois indépendamment H, un alkyle en C₁₋₁₂ éventuellement substitué, un aryle en C₆₋₁₂, un alkylaryle en C₇₋₁₃, un alcanoyle en C₁₋₁₂ éventuellement substitué, un aroyle en C₇₋₁₃ éventuellement substitué, un oligoéthylèneglycol ayant 1 à 6 atomes d'oxygène, un oligoéthylèneglycoléther ayant 1 à 6 atomes d'oxygène, un imidazoylméthyle ou un radical correspondant, dans lequel un atome d'azote est substitué par un radical alkyle en C₁₋₁₂ et est éventuellement chargé positivement et un groupement C-H peut être remplacé dans le cycle par un C-(alkyle en C₁₋₁₂), un 1-(lactame en C₄₋₆)méthyle, qui peut être substitué sur le cycle par un alkyle en C₁₋₁₂,
R⁷ représente chaque fois indépendamment H, un alkyle en C₁₋₁₂, un aryle en C₆₋₁₂ ainsi que leurs structures tautomères,
ou des complexes métalliques des composés cycliques ou des complexes des composés cycliques avec des acides minéraux,
dans lesquels, s'agissant de cycles cationiques, seront présents comme contre-ions X-, un chlorure, un sulfate, un hydrogénosulfate, un phosphate, un hydrogénophosphate, un nitrate, BF₄⁻ et un méthanesulfonate,
comme absorbeurs de lumière, matériaux pour couches d'injection de trous dans des diodes électroluminescentes organiques (OLED), comme composés électroluminescents dans des OLED, comme agents synergiques pour la dispersion de pigments ou pour le stockage optique de données.

2. Utilisation selon la revendication 1, **caractérisée en ce que** l'on utilise des composés cycliques de formule générale (I) 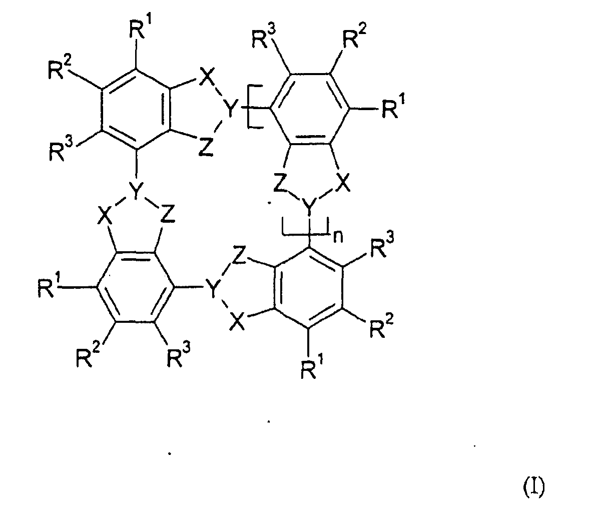 avec les significations suivantes :
n est un nombre dans la plage de 1 à 7,
X-Y-Z représente chaque fois indépendamment O-C=N, N=C-O, NH-C=N, N=C-NH, S-C=N, N=C-S,
R¹, R² et R³ représentent chaque fois indépendamment H ou un substituant issu du groupe constitué des groupements alkyle en C₁₋₁₂, alcanoyle en C₁₋₁₂, cycloalkyle en C₃₋₇, aryle en C₆₋₁₂, aralkyle en C₇₋₁₃, alkaryle en C₇₋₁₃, alcoxy en C₁₋₁₂, aryloxy en C₆₋₁₂, hydroxyalkyle en C₁₋₁₂, un hétérocycle, aroyle en C₆₋₁₂, qui peuvent être respectivement substitués, hydroxy, thiol, halogène, cyano, isocyano, nitro, ammonium, amino, phosphine, oxyde de phosphine, un acide sulfonique ou un de ses dérivés, un acide carboxylique ou un de ses dérivés, des dérivés de silicium,
dans laquelle R¹ et R² et/ou R² et R³ peuvent également former chaque fois, indépendamment l'un de l'autre, des systèmes cycliques condensés, éventuellement substitués, composés de 1 à 3 cycles, qui peuvent contenir des groupes d'hétéroatomes, ou qui peuvent former des groupements alkylène, éventuellement substitués, qui peuvent être interrompus par des groupes d'hétéroatomes,
dans laquelle, en moyenne, 0,01 à 12% des radicaux R¹, R² et R³ présents dans la molécule peuvent être différents de l'hydrogène,
ou des composés hétérocycliques correspondants, dans lesquels au moins un groupement -CR¹=, -CR²=, -CR³ est remplacé par -N=,
ou des complexes métalliques des composés cycliques,
comme absorbeurs de lumière, matériaux pour couches d'injection de trous dans des diodes électroluminescentes organiques (OLED),
ou comme agents synergiques pour disperser des pigments.

3. Utilisation selon la revendication 1 ou 2, **caractérisée en ce que** l'absorbeur de lumière est un absorbeur d'UV et/ou un absorbeur de lumière visible.

4. Utilisation selon l'une quelconque des revendications 1 à 3, **caractérisée en ce que** l'on utilise les composés cycliques de formule générale (I) dans un milieu d'application sous forme soluble, partiellement soluble ou insoluble, des cristaux mixtes pouvant également être présents avec d'autres colorants sous la forme insoluble.

5. Utilisation selon l'une quelconque des revendications 1 à 4, **caractérisée en ce que** R¹, R² et R³ ont les mêmes significations pour toutes les positions.

6. Composés cycliques ou complexes métalliques des composés cycliques ou complexes des composés cycliques avec des acides minéraux, comme définis dans la revendication 1 ou 2, à l'exception des composés cycliques ayant la signification suivante :
X-Y-Z représentant N=C-O, NH-C=N, N=C-NH,
R¹, R² et R³ représentant H ou un alkyle en C₁₋₆.

7. Procédé pour fabriquer des composés cycliques de formule générale (I) selon la revendication 6, par cyclisation de composés de formule générale (II) 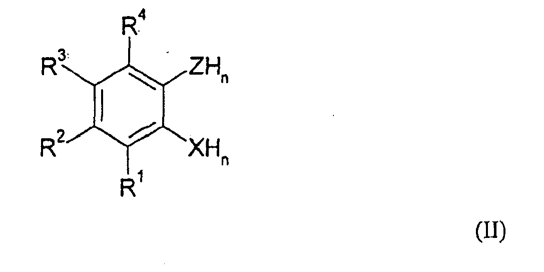 avec les significations suivantes :
R¹, R², R³, X et Z sont tels que précédemment indiqués,
R⁴ représente -COOH ou un dérivé de celui-ci,
n est chaque fois égal à 1 ou à 2 pour maintenir la stoechiométrie,
dans laquelle des groupements OH peuvent également être présents sous la forme d'un sel de métal alcalin ou d'un sel d'ammonium et/ou les groupements NH₂ peuvent se présenter sous une forme protonée ou une forme dérivée, telle que -NO, -NO₂, -N=N-aryle, =NOH, =NH, la cyclisation pouvant être effectuée en présence de sels métalliques, de poudres métalliques ou d'acides de Lewis comme matrices et en présence d'agents de condensation ou dans des conditions déshydratantes.

8. Procédé pour fabriquer des complexes de composés cycliques en fabriquant les composés cycliques selon un procédé de la revendication 7 en présence de sels métalliques, de poudres métalliques comme matrices ou en faisant réagir des composés cycliques selon la revendication 7 avec des sels métalliques ou des poudres métalliques.

9. Utilisation selon l'une quelconque des revendications 1 à 5 comme absorbeurs de lumière pour colorer des matériaux organiques de poids moléculaires élevés.

10. Masses de moulage, vernis et compositions de revêtement thermoplastiques contenant des absorbeurs de lumière, tels qu'ils sont définis dans l'une quelconque des revendications 1 à 5.
